# EUROPEAN PATENT APPLICATION

(11) **EP 4 549 946 A1**
(43) Date of publication of application: **07.05.2025**
(21) Application number: 23856231.8
(22) Date of filing: 20.06.2023
(51) Int. Cl.: G01N 33/68, G01N 27/62, C12P 21/06, G16B 35/00

(54) **METHOD FOR DETECTING PROTEIN HAVING CHANGES IN ENERGY STATE, OR AFFINITY OF LIGAND TO PROTEIN**

(30) Priority: 26.08.2022 CN 202211030145
(71) Applicant: Dalian Institute Of Chemical Physics, Chinese Academy Of Sciences, Liaoning 116023 (CN)
(72) Inventor: YE, Mingliang, Dalian, Liaoning 116023 (CN); LI, Kejia, Dalian, Liaoning 116023 (CN)
(74) Representative: K&L Gates LLP
(86) International application number: PCT/CN2023/101306
(87) International publication number: WO 2024/041139

(57) **Abstract**

Disclosed in the present invention is a method for detecting a protein having changes in an energy state, and affinity of a ligand to a protein. Specifically, after the energy state of a protein changes, its tolerance to proteolytic cleavage destruction changes. The structure of the protein in a low-energy state is also destroyed under a non-denaturation condition by using a large amount of enzymes, and small peptide fragments, which have molecular weight of less than 5 KDa and can be directly used for bottom-top mass spectrometry analysis, are directly generated. The method has extremely high sensitivity. Quantitative proteomics is used to find enzyme cleavage differential peptide fragments, and proteins to which the differential peptide fragments belong and the positions in the proteins are analyzed, so that a protein having changes in an energy state, and a change region can be determined in the whole proteome range. If the energy state of the protein changes due to addition of a ligand, the method can determine a binding protein and a binding region of the ligand; and the output of a quantitative result on the peptide fragment level further enables the method to determine the local affinity of binding of the ligand to the protein.

## Description

### FIELD OF THE INVENTION

The present invention belongs to the research field of functional proteomics (Proteomics category). Specifically, it involves detecting proteins or protein regions undergoing changes in energy states, identifying the binding proteins of a ligand or determining the local affinities between a ligand and proteins in complex protein mixtures such as cell or tissue lysates.

### BACKGROUND OF THE INVENTION

Proteins are the primary players in biological processes. Traditional quantitative proteomics is commonly used to assess differences in protein abundance among different samples. However, many diseases, such as Parkinson's syndrome and Alzheimer's disease, as well as ligands (including drugs and endogenous metabolites) interacting with proteins, result in changes not in the abundance of specific proteins but rather in the protein folding status, post-translational modifications, or alterations in protein function (De Souza N, et al., Curr Opin Struct Biol, 2020, 60:57-65; Cappelletti V, et al., Cell, 2021, 184(2):545-559). These changes in protein properties beyond abundance can affect the thermodynamic stability, that is, the energy states of proteins. Therefore, the development of methods capable of detecting changes in the energy states of proteins is of significant importance in fields such as disease diagnosis, analysis of metabolic molecular pathways, and drug target discovery.

Top-down mass spectrometry analysis enables direct analysis of intact proteins, allowing for monitoring changes in the energy states of proteins (Sheng Y et al., International Journal of Mass Spectrometry, 2010, 300 (2011) 118-122). However, this method has low throughput and is mainly applicable to purified proteins, making it challenging to apply in complex protein mixtures. There are also non-mass spectrometry methods available to detect changes in the energy states of proteins, such as fluorescence resonance energy transfer (Heyduk T, et al., Curr. Opin. Biotechnol, 2002, 13, 292-296) and nuclear magnetic resonance (Sakakibara D, et al., Nature, 2009, 458, 102-105). These techniques are suitable for purified proteins and are therefore not applicable at the proteomic level.

Bottom-up mass spectrometry is a technique that involves digesting large protein fragments into smaller peptides for subsequent mass spectrometry analysis. This technique allows for the identification or quantification of thousands, or even tens of thousands of proteins simultaneously. In recent years, a series of high-throughput methods combining bottom-up mass spectrometry techniques to analyze changes in protein energy states have received widespread attention. These methods are based on the altered tolerance to heat or denaturants following changes in protein energy states. Consequently, proteins with lower energy states are more likely to maintain their original conformation, making them less prone to precipitation or structural unfolding when subjected to the same intensity of thermal or denaturant stimuli. By analyzing the differences in the amount of precipitated or unfolded proteins between two or more groups of samples, proteins undergoing changes in energy states can be identified. Such techniques include Cellular Thermal Shift Assay (CETSA, Martinez Molina et al., Science, 2013, 341(6141):84-87), Thermal Proteome Profiling (TPP, Savitski M et al., Science, 2014, 346(6205):55-+), and Pulse Proteolysis (PP, Chiwook P et al., Nature Methods, 2005, 2(3):207-212), etc. These methods for detecting changes in protein energy states have successfully been applied to identify drug target proteins (Savitski M et al., Science, 2014, 346(6205):55-+; Perrin J et al., Nature Biotechnology, 2020, 38(3):303-308), analyze protein-protein interactions (Tan C S et al., Science, 2018, 359(6380):1170-1177), and study functional post-translational modifications (Huang J X et al., Nature Methods, 2019, 16, 894-901). However, these methods analyze changes in protein energy states at the protein level and cannot determine the specific regions where protein energy states undergo changes. In addition, for proteins with multiple domains, changes in local stability may have minimal impact on the overall stability of the protein, making it difficult to capture stability changes at the protein level (Mateus A, et al., Proteome Sci, 2016, 15:13).

Limited Proteolysis (LiP) has long been used to study protein conformation. This method is based on the differential susceptibility of a protein to proteolysis in different conformations. By using a small amount of nonspecific protease, the protein is slightly cleaved, primarily targeting the disordered regions in high energy states and generating large protein fragments. When the protein undergoes conformational changes (e.g., increased rigidity in disordered regions), the susceptibility to proteolysis at the conformationally changed region also changes. The differential abundance of the resulting protein fragments, determined through SDS-PAGE, identifies the regions of protein conformational changes. Since changes in protein energy states often accompany conformational changes, this method can also be used to study changes in protein energy states and identify the regions where protein energy states change (Antonio Aceto, et al., The International Journal of Biochemistry & Cell Biology, 1995, 27(10):1033-1041; Polverino de Laureto, et al., J Mol Biol, 2003, 334(1):129-141). However, due to the difficulty of directly analyzing large protein fragments in a bottom-up mass spectrometry analysis mode for quantification, this technique is limited to the characterization of purified proteins.

Paola et al. combined Limited Proteolysis (LiP) with bottom-up mass spectrometry analysis (Feng YH, et al., Nature Biotechnology, 2014, 32(10):1036-+): they subjected the large fragments generated from limited proteolysis (i.e., the aforementioned large protein fragments) to denaturation using denaturing agents; they further performed complete trypsin digestion of the denatured protein fragments to generate semi-tryptic peptides containing a nonspecific cleavage site and fully tryptic peptides without a nonspecific cleavage site. By quantifying the abundance differences of semi-tryptic or fully tryptic peptides in two or more groups of samples using bottom-up quantitative proteomics, they determined the proteins undergoing conformational changes. Additionally, by analyzing the positions of these peptides on the protein, they determined the regions where conformational changes occurred. This method has to some extent achieved proteome-level analysis of protein energy state changes and captured interactions between metabolites and proteins in relatively simple biological systems (Piazza I, et al., Cell, 2018, 172(1-2):358-372) as well as protein structural changes due to post-translational modifications, heat stimulation, and osmotic pressure (Cappelletti V, et al., Cell, 2021, 184(2):545-559). However, the two-step digestion in this method significantly increased the complexity of the samples and could generate interfering peptides. Therefore, this method is mostly applied in relatively simple biological systems. Paola et al. (2020) further developed LiP-Quant (Piazza I, et al., Nature Communications, 2020, 11(1):4200), a method that improves the reliability of target protein identification of LiP-MS by incorporating a machine learning algorithm that requires multiple ligand concentrations. LiP-Quant has to some extent achieved the identification of ligand-binding proteins and their binding regions in mammalian cell lysates. However, due to complex peptide mixtures generated from the two-step digestion, it is challenging to identify semi-tryptic peptides that contain the initial unspecific proteolytic sites. Additionally, peptides with an unspecific proteolytic site exhibit a smaller response to ligand binding, resulting in very limited sensitivity for the identification of ligand-binding target proteins. For instance, in the identification of target proteins for the broad-spectrum kinase inhibitor staurosporine, LiP-Quant could only identify 9 kinase target proteins in HeLa cell lysates when requiring that >80% of all identified candidate target proteins are kinases (Piazza I, et al., Nature Communications, 2020, 11(1):4200). In contrast, the present invention identified 111 kinase target proteins in HeLa cell lysates (see Example 3).

The aforementioned proteolysis-based methods involve using a small amount of protease to cleave the protein, resulting in cleavage sites located in disordered regions of high energy states. Different conformations of the protein generate distinct protein fragments. The conformational changes in the protein are determined by detecting the differences in the generation of protein fragments through SDS-PAGE or secondary digestion assisted by denaturing agents. There is no hint of using a larger amount of protease to induce disruptive digestion of the proteins (i.e., structures in the high-energy states of proteins are first digested; this induces the exposure of proteolytic sites in low-energy states; with a substantial amount of protease present, structures in low-energy states are also digested) to directly generate small peptides with a molecular weight less than 5 kDa for bottom-up mass spectrometry analysis. These peptides feature two cleavage sites reflecting the stability of protein regions. In the bottom-up mass spectrometry analysis mode, the method quantitatively analyzes the differential abundance of these small peptides to determine the proteins and protein regions that undergo changes in energy states and ligand-protein binding affinities. We have invented a method that can sensitively determine the proteins and protein regions that undergo changes in energy states, as well as measure the local affinities between ligands and proteins in complex protein mixtures.

This method utilizes disruptive digestion to directly generate small peptides that are suitable for bottom-up mass spectrometry analysis and contain two cleavage sites both reflecting proteins' local stabilities. Compared to the complex peptide mixtures obtained from two-step digestion with two proteases in LiP-MS, this method significantly reduces sample complexity. Furthermore, the generation of these peptides involves a cascading-like multi-stage digestion process (i.e., protein structures in high energy states are initially disrupted, inducing further digestion of protein structures in low energy states). Within this cascading process, the cumulative differences in digestion rates at each stage result in small peptides exhibiting large magnitudes of responses to the changes in protein energy states or binding with ligands. Therefore, this method exhibits high sensitivity in detecting changes in protein energy states. For example, compared to LiP-Quant, when requiring the kinase proportion to be greater than 80% among all identified staurosporine target proteins, this method can identify 111 kinases in HeLa cell lysates, which is a 12.3-fold improvement over LiP-Quant's identification of 9 kinases (see Example 3). This method provides quantitative results on a peptide level and can be used to calculate the local binding affinities between protein regions and ligands when using multiple ligand doses.

### DETAILED DESCRIPTION OF THE INVENTION

The purpose of the present invention is to develop a method that can sensitively determine proteins or protein regions that undergo changes in energy states, as well as can determine the local affinity of ligands to proteins. This method is referred to as PEptide-centric Local Stability Assay (PELSA).

The principle behind this invention is that when the energy state of a protein changes, its local or global stability also changes, altering its ability to resist external disruption. Proteolysis is a disruptive process. When protein energy state changes, the resistance to proteolysis is altered. At high concentrations of protease, the protein is first cleaved into large fragments. Due to the disruptive nature of proteolysis, the protein structures in a high-energy state or without ligand binding are disrupted and quickly unfold, inducing further proteolysis of protein structures in the low energy states and ultimately producing small peptides (< 5 kDa) that are suitable for bottom-up mass spectrometry analysis and indicative of protein's local stability. On the other hand, the protein region in a low-energy state or with ligand binding maintains a stable structure and is less likely to enter the active pocket of the protease, making it difficult to generate small peptides. By comparing the abundance differences of such peptides, proteins with altered energy states can be identified. Analyzing the locations of different peptides on the protein can determine the protein regions with altered energy states. In this method, proteins directly generate small peptides containing two cleavage sites that both reflect protein's local stability under non-denaturing conditions. Due to the cascading digestion process of generating small peptides, the difference in proteolytic rates of every stage accumulates which leads to the large magnitudes of responses to the changes in protein energy state or binding with ligands. Furthermore, since this method employs extensive disruptive digestion of the proteins, the cleavage sites are not limited to disordered regions of high energy states, resulting in a greater number of peptides that reflect changes in protein stability. Additionally, using only one protease in this process significantly reduces the complexity of the generated peptides, allowing for interrogating a large number of proteins. These characteristics make this method highly sensitive.

When a ligand is exposed to a complex protein sample, the binding of the ligand with the target proteins will result in the change of the energy states of the binding regions in target proteins. Therefore, the binding target proteins and the binding regions could be determined by analyzing the changes of the energy states for all proteins in the sample at the proteome level with this invented method.

The principle of this technique for determining the local affinity of ligands to proteins is based on the dose-dependent changes in the local stability of the target protein with respect to the dose of the added ligand, as long as saturation concentration is not reached. By detecting the abundance of cleaved peptides in samples treated with different ligand concentrations, the local affinity of the ligand to the protein region can be calculated.

The detected peptides possess two cleavage sites that both reflect the protein local stability. Different stability levels of various regions in the protein under its tested conformation result in varying degrees of susceptibility in proteolysis. The cleavage sites generated under such conditions are referred to as cleavage sites reflecting the protein local stability.

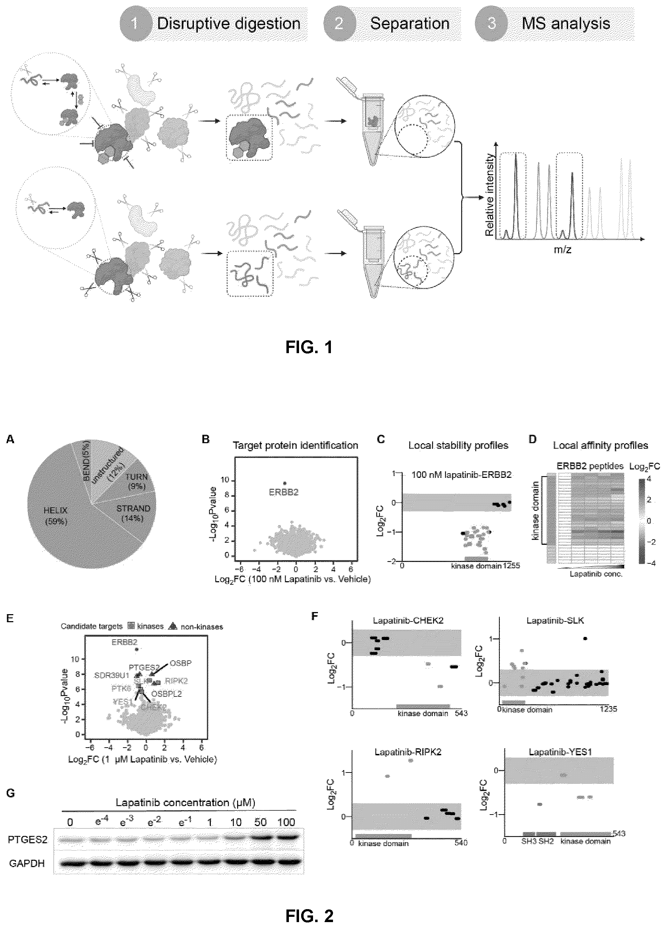

The present invention employs the following technical scheme (FIG. 1): (a) Proteins in different energy states or proteins incubated with ligands of varying concentrations are prepared, and then disruptive proteolysis is performed using a specific ratio of protease to protein, allowing most proteins to generate small peptides with a molecular weight less than 5 kDa. These peptides contain two cleavage sites both reflecting the protein's local stability. (b) The cleaved peptides are isolated and quantitatively analyzed using a bottom-up mass spectrometry analysis mode. (c) The differences in abundance of the cleaved peptides are analyzed to determine the proteins and protein regions undergoing changes in energy state, as well as the local affinity of ligands to the protein.

Specifically, it includes the following steps (as an example):
1) The proteins mentioned in step (a) should retain their conformation for testing. If tissue or cell lysate is obtained through lysis, gentle lysis methods should be employed such as repeated freezing and thawing with liquid nitrogen, liquid nitrogen grinding, or homogenization. The lysis solution used should also preserve the protein's conformation for testing.
2) During the investigation of ligand-protein binding, the biological protein samples are divided into two groups. The experimental group is treated with a specific concentration of the ligand, while the control group is treated with a blank solvent. However, it is not limited to only two groups; the experimental group can also include multiple groups with varying concentrations of ligands.
3) Protease is added to the protein sample by weight ratio of protease to total protein ranging from 1:1 to 1:50. Digestion is performed at a certain temperature for 0.5 min to 60 min, using an oscillating shaker at a speed of 1000 rpm to 1500 rpm.
4) The digested samples from step 3) are heated at 100 °C in a metal bath for 5 min to terminate the digestion.
5) The heating-induced precipitates are dissolved in 6 M guanidine hydrochloride (final concentration) followed by adding a final concentration of 10 mM tris(2-carboxyethyl)phosphine (TCEP) and a final concentration of 40 mM chloroacetamide (CAA) at 95 °C for 5 min to alkylate the post-digestion samples.
6) The alkylated samples from step 5) are transferred to a 10 kDa ultrafiltration unit and centrifuged at 14000 g for 20 min to 1 h to isolate the small peptides. Subsequently, the ultrafiltration membrane is washed with 200 µL pH 8.2 HEPES buffer followed by another centrifugation at 14000 g to isolate the residual peptides. The residual peptides are combined with the peptides collected in the initial centrifugation cycle.
7) The collected peptides from step 6) are quantified using mass spectrometry, utilizing either label-based or label-free quantification methods.
8) The quantification results are analyzed at the peptide level, with the significance of or fold change of the first/second/third/fourth -rank peptide (ranking by significance or fold change) are used as indicators for the significance or fold change of the protein. This approach is employed to identify reliable target proteins.
9) The peptides are assigned to their respective protein sequences or structures to identify the protein regions undergoing changes in energy state. The fold changes of the peptides at different ligand concentrations and the corresponding ligand concentrations are fitted into a four-parameter logistic equation (Y=Bottom+(Top-Bottom)/(1+10^(LogEC50-X)*Hill Slope)). This calculation enables the determination of the local affinity between the ligand and the protein, as represented by the EC50 value in the equation.

Compared to existing methods, the present invention, known as PELSA, exhibits several innovations and advantages:
(1) PELSA explores the concept that proteolysis is a disruptive process. By using a large amount of protease, proteins under non-denaturing conditions are initially disrupted at the protein structures of high energy states with digestion, which leads to the exposure of proteolytic sites at the protein structures of low energy states. In the presence of large amount of protease, the protein structures in the low energy states are also digested and disrupted, allowing most proteins to directly generate small peptides with a molecular weight less than 5 kDa. These peptides are then identified and quantified using mass spectrometry. The peptides identified in PELSA are generated from the cascading-like disruptive digestion process and these peptides can be directly analyzed by mass spectrometry in a bottom-up manner. When proteins undergo changes in energy states or bind with ligands, the cumulative difference in proteolytic rates at each stage of the cascading digestion process result in significant responses from the peptides. Additionally, this method utilizes a large amount of protease, enabling the generation of a greater number of ligand-responsive peptides. For example, in Example 2, PELSA identified 2-5 times more ligand-responsive peptides compared to the LiP-MS method. Furthermore, the fold change magnitudes of these peptides measured in PELSA were 4-6 times higher than those observed in LiP-MS.
(2) Wide applicability: In comparison to traditional methods based on affinity chromatography, the method bypasses the need for chemical modification of ligand molecules when it is used to identify ligand-binding proteins. This means that the method can, in theory, be applied to any ligand. Additionally, the identification of ligand-binding target proteins is not dependent on the strength of their binding affinity, making it suitable for the identification of target proteins with low ligand affinity. For instance, successful identification of the target proteins of metabolites such as folate, leucine, and α-ketoglutarate (αKG) is demonstrated in Examples 4-6. Notably, Example 6 represents the largest number of known αKG target proteins identified in a single experiment to date.
(3) Determination of protein regions with different energy states or binding regions of ligands: In comparison to the widely-applied Thermal Proteome Profiling (TPP) method, this approach enables the identification of regions within proteins that undergo changes in energy states or regions involved in ligand binding. This determination is achieved by analyzing the positions of the altered peptides within the proteins. As demonstrated in Example 3, this method successfully identified the binding regions of the kinase inhibitor staurosporine on 154 kinases (total number of kinase targets identified in HeLa and K562 cell lysates).
(4) High sensitivity: Unlike protein-level readout methods such as TPP that averages local changes in protein energy states on the whole protein, this method directly captures the local changes at the peptide level. Consequently, it exhibits greater sensitivity in detecting subtle local changes. Compared with existing peptide-level readout method such as LiP-MS, the determination of ligand binding regions does not rely on identifying peptides with specific characteristics such as those containing non-specific cleavage sites as required in LiP-Quant. This makes it easier to identify differential peptides and the target proteins. For instance, in Example 3, PELSA identified a 2.1-fold increase in the number of kinase target proteins for the broad-spectrum kinase inhibitor staurosporine compared to TPP, and a remarkable 12.3-fold increase compared to LiP-Quant.
(5) Evaluation of local ligand-protein binding affinity: The protein samples are divided into multiple groups, and different concentrations of ligands are added to each group. As the ligand concentration varies, the susceptibility of the target protein to proteolysis changes, reflected by the change in abundance of the generated peptides. By fitting a four-parameter logistic equation to the data, with ligand concentration as the x-axis and the fold change in peptide abundance as the y-axis, the affinity information can be obtained. Each identified protein may have multiple peptides, and the affinity curves of these peptides represent the local binding affinities of the ligand to specific regions. Example 11 demonstrated that the local affinities measured by PELSA are very close to those determined using microscale thermophoresis assay.
(6) Broad application prospects: Protein-ligand binding, protein post-translational modifications, protein structural changes, and protein-protein interactions can all induce alterations in protein energy states. Therefore, these biological events could all be investigated by this method. Example 7 demonstrates the application of PELSA in analyzing changes in protein energy states caused by protein-protein interactions. Example 8 showcases its use in analyzing changes in protein energy states resulting from binding with post-translationally modified peptides. Example 9 illustrates PELSA's effectiveness in analyzing changes in protein energy states induced by metal-ion binding.

### BRIEF DESCRIPTION OF THE FIGURES

FIG. 1 PELSA Workflow: Proteins with different energy states (e.g., a protein in the ligand-bound state and the same protein in the ligand-unbound state) undergo disruptive trypsinization in a specific ratio of protease to substrate. Due to the binding of the ligand to the protein, the binding region becomes more stable, resulting in less susceptibility to unfolding and thus generation of less mount of peptides compared to the control group. The digestion products are subjected to denaturation and alkylation, followed by ultrafiltration to isolate the generated peptides for quantitative mass spectrometry analysis.
FIG. 2 Application of PELSA in analyzing changes in protein energy states in cell lysates following treatment with the anti-breast cancer drug lapatinib. (A) Analysis of the protein secondary structures of PELSA cleavage sites: PELSA cleavage sites refer to the N- and C-terminal residues of all identified peptides in the PELSA experiment. Protein secondary structure information is sourced from AlphaFold, and the classification of secondary structures is based on the literature (Bludau I, et al, PLoS Biol, 2022, 20(5): e3001636). HELIX represents low-energy state helical structures, STRAND represents folded structures, BEND indicates bent structures, TURN signifies turn structures, and unstructured denotes high-energy state disordered structures. (B) Protein-level volcano plot corresponding to 100 nM lapatinib-treated BT474 cell lysates: BT474 cell lysates were treated with 100 nM lapatinib or DMSO (in four replicates). Fold change and P-value of each peptide was calculated using Empirical Bayes t-test. The peptide with the smallest P-value per protein was used to represent the corresponding protein, and the log2 fold change and -log10 Pvalue of the protein are plotted. (C) Two-dimensional local stability profiles of ERBB2: Log2 fold change of quantified peptides of ERBB2 and their corresponding positions on the two-dimensional sequence of ERBB2. (D) Local affinity profile of ERBB2: Log2 fold change of ERBB2 peptides at different concentrations of lapatinib. (E) Protein-level volcano plot corresponding to 1 µM lapatinib-treated BT474 cell lysates. (F) Two-dimensional local stability profiles of off-target kinases identified for lapatinib, using CHEK2, SLK, RIPK2, and YES1 as examples. (G) Western blotting confirms the stabilization of PTGES2 upon lapatinib treatment.
   Unless otherwise stated, the terms "fold change", "FC" (short for fold change), or "ratio" in this specification refer to the ratio of peptide abundance between the experimental and control groups.

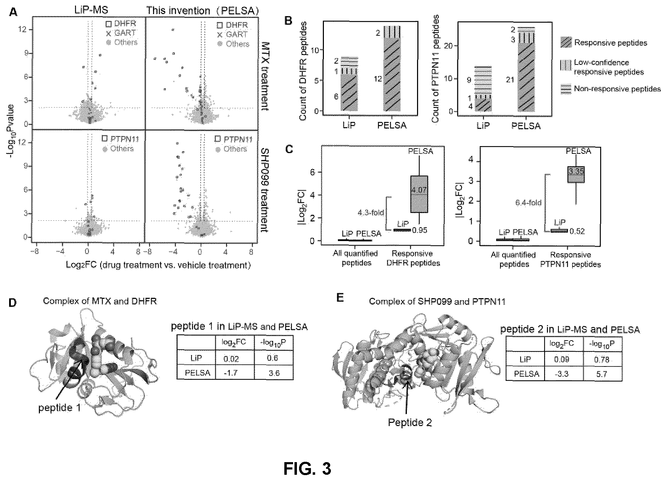

FIG.3 Comparison of PELSA and LiP-MS for the identification of proteins undergoing changes in energy states in HeLa cell lysates treated with methotrexate (MTX) or SHP099. (A) (Top) Peptide-level volcano plots obtained from LiP-MS/PELSA for HeLa cell lysates treated with 10 µM MTX. (Bottom) Peptide-level volcano plots obtained from LiP-MS/PELSA for HeLa cell lysates treated with 10 µM SHP099. (B) PELSA identified a higher number of ligand-responsive peptides for DHFR and PTPN11 compared to LiP-MS, with 2.00-fold and 5.25-fold increases, respectively. (C) The magnitudes of log2 fold changes of ligand-responsive DHFR and PTPN11 peptides identified by PELSA were significantly larger, with 4.3-fold and 6.4-fold increases, respectively, compared to those identified by LiP-MS. (D) The PDB structure of DHFR bound to MTX, with an arrow indicating a peptide located at the MTX-binding site within a low-energy state helical region. This peptide exhibited significant fold changes in PELSA but remained unchanged in LiP-MS; (E) The PDB structure of PTPN11 bound to SHP099, with an arrow indicating a peptide situated at the SHP099-binding regions and embedded within the protein structure. Similarly, this peptide showed significant fold changes in PELSA but no fold changes in LiP-MS.

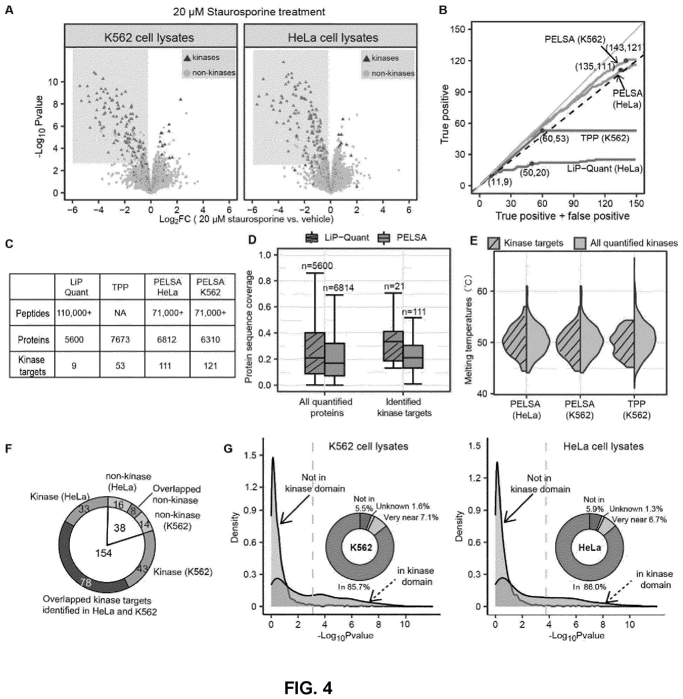

FIG.4 demonstrates the high sensitivity of PELSA in identifying proteins undergoing changes in energy state, using the example of screening for target proteins of a pan-kinase inhibitor staurosporine. (A) Protein-level volcano plots corresponding to 20 µM staurosporine-treated K562 cell lysates (left) and HeLa cell lysates (right). (B) A comparison of the number of staurosporine target proteins identified by PELSA in K562 cell lysates, PELSA in HeLa cell lysates, LiP-Quant in HeLa cell lysates as previously reported, and TPP in K562 cell lysates as previously reported. The x-axis represents the total number of identified target proteins, and the y-axis represents the number of kinases among the identified targets. (C) The number of proteins and peptides, and the final number of kinase targets identified by LiP-Quant (HeLa), TPP (K562), PELSA (K562), and PELSA (HeLa). (D) A comparison of protein sequence coverages for all proteins identified by PELSA (HeLa) and LiP-Quant (HeLa) (left), and the comparison of protein sequence coverages for kinase targets identified by PELSA (HeLa) and LiP-Quant (HeLa) (right). This graph indicates that PELSA can identify target proteins with lower protein sequence coverages. (E) Split violin plots illustrating the distribution of thermal melting points for kinase targets identified by PELSA (HeLa), PELSA (K562), TPP (K562), and for all quantified kinase proteins in each dataset. This graph demonstrates that PELSA is effective in identifying both thermo-resistant and thermo-sensitive target proteins. (F) Overlap of kinase targets identified by PELSA (K562) and PELSA (HeLa). (G) Density plots showing the -log10Pvaue of peptides within kinase domains and those out of kinase domains in PELSA-K562 experiment (left) and PELSA-HeLa experiment (right). The x-axis represents - log10Pvalue calculated using the Empirical Bayes t-test, and the y-axis represents the density. The circular plot indicates the locations of peptides that show significant fold changes by staurosporine treatment. Notably, the majority of peptides with significant fold changes are located within the kinase domains.

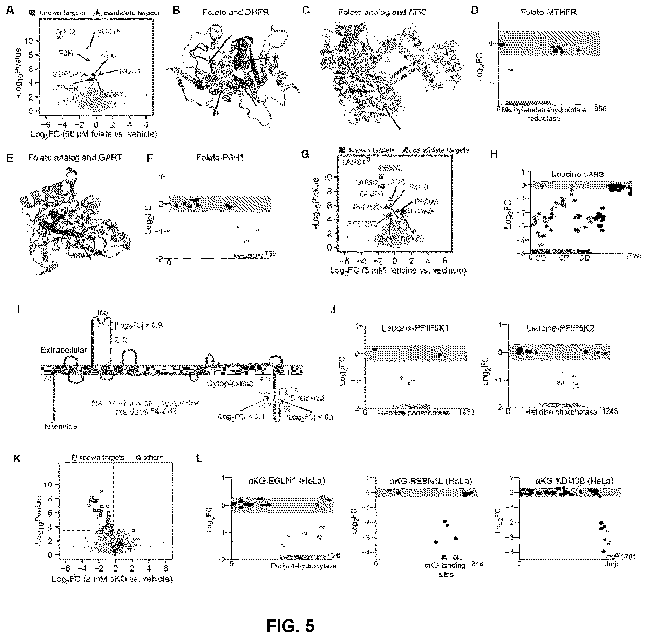

FIG.5 illustrates the application of PELSA in analyzing protein local energy state changes in HeLa cell lysate upon metabolite treatment. (A) Protein-level volcano plot corresponding to 50 µM folate-treated K562 cell lysate. (B) Three-dimensional structure plot demonstrating local stability changes of DHFR upon folate binding as measured by PELSA, with an arrow indicating the protein segment with the largest stability change. (C) Three-dimensional structure plot demonstrating local stability changes of ATIC upon addition of 50 µM folate as measured by PELSA, with an arrow indicating the protein segment with the largest stability change. (D) Two-dimensional protein sequence plot demonstrating local stability changes of MTHFR upon addition of 50 µM folate as measured by PELSA. (E) Three-dimensional structure plot demonstrating local stability changes of GART upon addition of 50 µM folate as measured by PELSA, with an arrow indicating the protein segment with the largest stability change. (F) Two-dimensional protein sequence plot demonstrating local stability changes of P3H1 upon addition of 50 µM folate as measured by PELSA. (G) Protein-level volcano plot corresponding to 5 mM leucine-treated K562 cell lysates. (H) Two-dimensional protein sequence plot demonstrating local stability changes of LARS1 upon addition of 5 mM leucine as measured by PELSA. (I) Topological structure diagram of the membrane protein SLC1A5, with the start and end positions of three quantified peptides (190-212, 493-502, and 523-541) indicated, along with their corresponding |log2FC| values. (J) Two-dimensional protein sequence plot demonstrating local stability changes of PPIP5K1 and PPIP5K2 upon addition of 5 mM leucine as measured by PELSA. (K) Protein-level volcano plot corresponding to 2 mM α-ketoglutarate (αKG)-treated HeLa cell lysates, with the dashed lines representing -log10P value = 3.4 and log2FC = -0.5. Among the 40 proteins meeting the criteria of -log10Pvalue > 3.4 and log2FC < -0.5, 30 are known target proteins of αKG. (L) Two-dimensional protein sequence plot demonstrating local stability changes of EGLN1, RSBN1L, and KDM3B upon the treatment of HeLa cell lysates with 2 mM αKG. The protein segments detected to have altered energy states by PELSA are known αKG-binding regions.

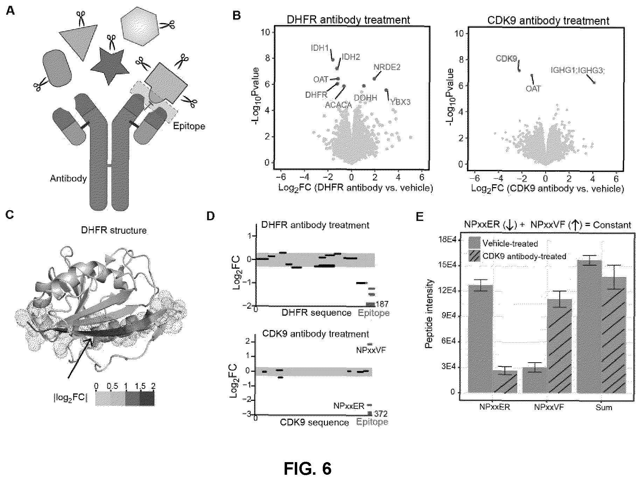

FIG.6 depicts the application of PELSA in analyzing protein local energy state changes in HeLa cell lysates resulting from protein-protein interactions (using antibody-antigen binding as an example). (A) Schematic representation of PELSA identifying antibody-binding epitopes in cell lysate. (B) (Left) Protein-level volcano plot corresponding to DHFR antibody-treated HeLa cell lysate. (Right) Protein-level volcano plot corresponding to CDK9 antibody-treated HeLa cell lysates. (C) Three-dimensional structure plot demonstrating local stability changes of DHFR upon addition of DHFR antibody as measured by PELSA (PDB:1BOZ), with shaded spheres indicating the antibody binding epitope, and the arrow indicating the protein segment with the greatest stability change. (D) (Top) Two-dimensional protein sequence plot demonstrating local stability changes of DHFR upon addition of DHFR antibody as measured by PELSA. (Bottom) Two-dimensional protein sequence plot demonstrating local stability changes of CDK9 upon addition of CDK9 antibody as measured by PELSA. (E) Abundance changes of the two peptides, NPATTNQTEFERVF and NPATTNQTEFER, of CDK9 upon addition of CDK9 antibody.

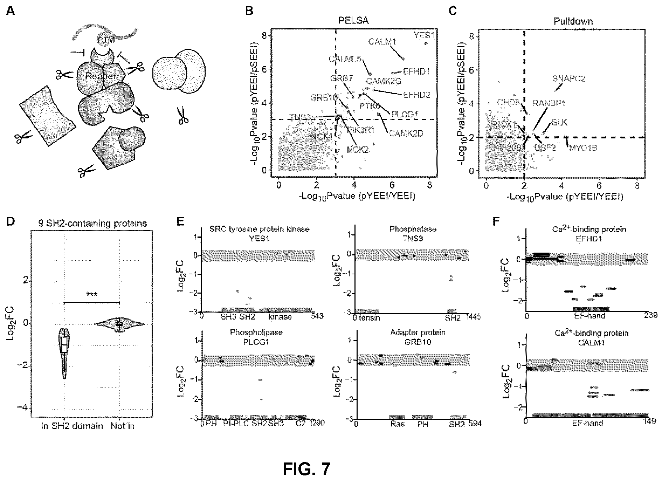

FIG.7 depicts the application of PELSA in analyzing protein local energy state changes in BT474 cell lysates resulting from binding with post-translationally modified peptides (illustrated by identifying the recognition domains of a phosphorylated peptide). (A) Schematic representation of PELSA identifying recognition domains of a post-translational modification in cell lysates. (B) Protein-level scatter plot in the PELSA experiment, where the peptide with the highest sum of -log10Pvalue (pYEEI/pSEEI) and -log10Pvalue (pYEEI/YEEI) is used to represent the corresponding protein. The x-axis represents the -log10Pvalue (pYEEI/pSEEI) of the peptide with the highest sum of -log10Pvalues among all identified peptides for a given protein, and the y-axis represents the -log10Pvalue (pYEEI/YEEI) of the same peptide. (C) Protein-level scatter plot in Pulldown experiment. The x-axis represents the -log10Pvalue (pYEEI/pSEEI) and the y-axis represents -log10Pvalue (pYEEI/YEEI) for each protein. (D) Violin plots displaying the log2FC distribution of peptides within and outside the SH2 domains of 9 SH2 domain-containing target proteins identified by PELSA. (E) Two-dimensional protein sequence plots demonstrating local stability changes of SH2 domain-containing proteins identified by PELSA, showcasing examples such as YESI, TNS3, PLCG1, and GRB10. (F) Two-dimensional protein sequence plots demonstrating local stability changes of calcium-binding proteins identified by PELSA, showcasing examples such as EFHD1 and CALM1.

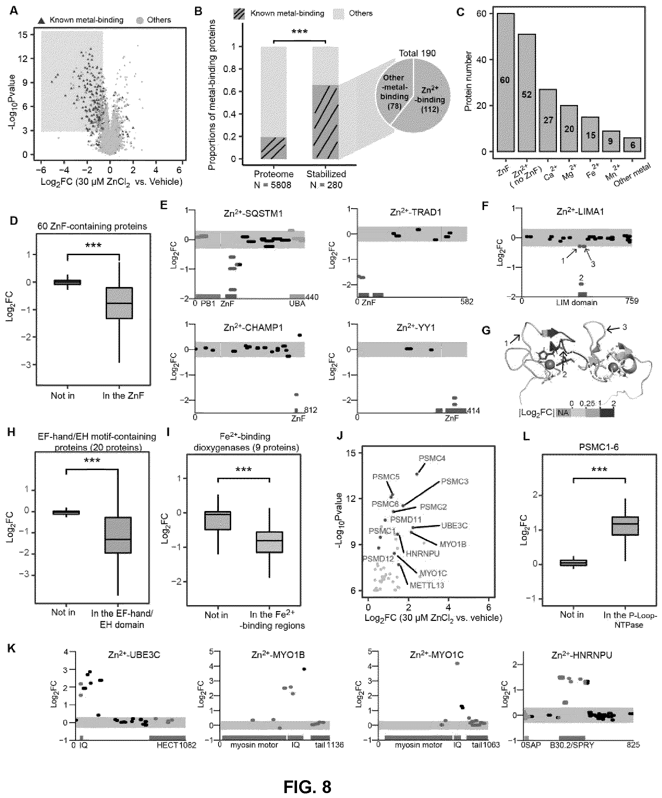

FIG.8 depicts the application of PELSA in analyzing protein local energy state changes in HeLa cell lysates resulting from binding with metal ions (illustrated with zinc ions) (A) Protein-level volcano plot corresponding to 30 µM ZnCl2-treated HeLa cell lysates. (B) Proportions of metal ion-binding proteins among all identified proteins (left) and among the 280 target proteins determined by PELSA (right). The pie chart on the right illustrates the ratio of zinc ion-binding proteins among the metal ion-binding proteins identified as target proteins by PELSA. (C) Bar graph showing the count of distinct metal ion-binding proteins among the metal ion-binding proteins identified as target proteins by PELSA. (D) Distribution of log2 fold change for peptides derived from 60 PELSA-identified target proteins containing zinc finger motifs, grouped based on peptides located within and outside the zinc finger motifs. (E) Two-dimensional protein sequence plots demonstrating local stability changes of PELSA-identified target proteins containing zinc finger motifs, showcasing examples such as SQSTM1, TRAD1, CHAMP1, and YY1. (F) Two-dimensional protein sequence plots demonstrating local stability changes of a zinc ion-binding protein LIMA1, which lacks zinc finger motifs. Three peptides within the LIM domain are labeled as 1, 2, and 3. (G) Three-dimensional structure of LIM domain of LIMA1, with zinc ions represented as spheres. The labeling of the three peptides within the LIM domain is same to that in (F). (H) Distribution of log2 fold changes for peptides derived from 20 PELSA-identified target proteins containing EF-hand/EH motifs, grouped based on peptides located within and outside the EF-hand/EH motifs. (I) Distribution of log2 fold changes for peptides derived from 9 PELSA-identified target proteins containing Fe2+-binding domains, grouped based on peptides located within and outside the Fe2+-binding domains. (J) Zoom-in view of protein-level volcano plot of 30 µM ZnCl2-treated HeLa cell lysate, focusing only on proteins with increased stability (-log10Pvalue > 6, log2 fold change > 0). (K) Two-dimensional protein sequence plots demonstrating protein local stability changes of proteins containing IQ motifs (UBE3C, MYO1B, MYO1C) and proteins containing B30.2SPRY domains (HNRNPU) after the addition of 30 µM ZnCl2. (L) Distribution of log2 fold changes for peptides derived from PSMC1-6, grouped based on peptides located within and outside the P-loop-NTPase domains.

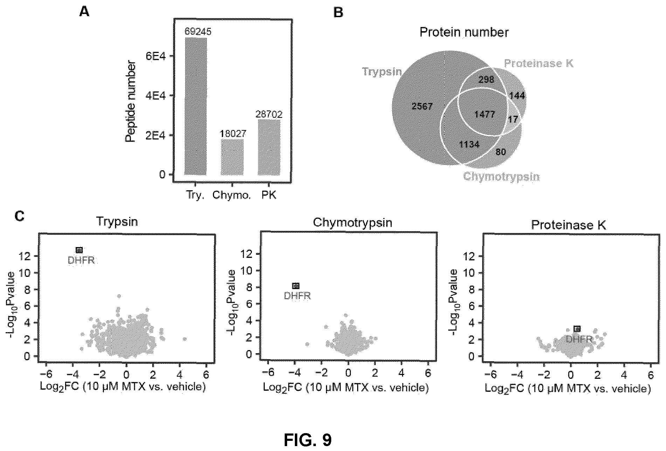

FIG.9 compares the protein coverages and performances of PELSA using different proteases. (A) Comparison of peptide identification numbers for Trypsin-PELSA, Chymotrypsin-PELSA, and Proteinase K-PELSA. (B) Venn diagram showing the overlap of identified proteins between Trypsin-PELSA, Chymotrypsin-PELSA, and Proteinase K-PELSA. (C) Protein-level volcano plots for Trypsin-PELSA (left), Chymotrypsin-PELSA (middle), and Proteinase K-PELSA (right) in HeLa cell lysate treated with 20 µM methotrexate (MTX).

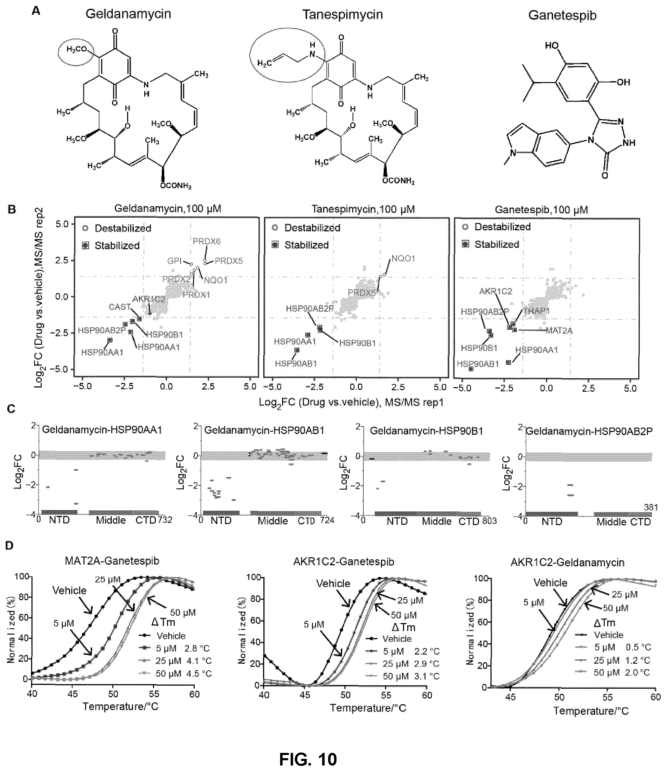

FIG.10 demonstrates the performance of dimethyl labeling-based PELSA in identifying proteins with altered energy states induced by HSP90 inhibitor binding. (A) Structures of the three heat shock protein inhibitors: geldanamycin (left), tanespimycin (middle), and ganetespib (right). (B) Protein-level scatter plots were generated for HeLa cell lysates treated with 100 µM geldanamycin, 100 µM tanespimycin, or 100 µM ganetespib. Each point in the plot represents a protein and is based on the peptide with the second largest absolute value of fold change among all identified peptides for that protein. The x-axis and y-axis of the plots depict the log2 fold changes obtained from two independent replicates of mass spectrometry analysis. (C) Two-dimensional protein sequence plots demonstrating protein local stability changes of HSP90AA1, HSP90AB1, HSP90B1, and HSP90AB2P, using geldanamycin treatment as an example. (D) Validation of the interactions between MAT2A and ganetespib, AKR1C2 and ganetespib, and AKR1C2 and geldanamycin using thermal shift assay.

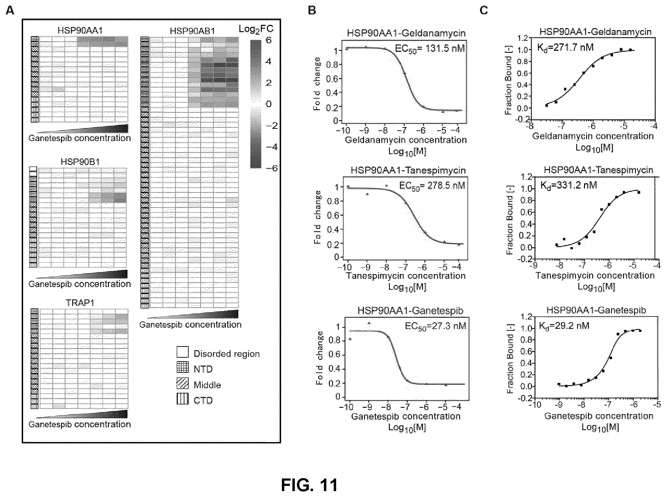

FIG.11 evaluates the local affinity between ligands and proteins using PELSA. (A) Fold changes in peptide abundance (ganetespib-treated vs. vehicle-treated) for heat shock proteins HSP90AA1, HSP90AB1, HSP90B1, and TRAP1 at different concentrations of ganetespib. (B) Affinity measurements between the HSP90AA1 and three inhibitors calculated by PELSA. The x-axis represents different inhibitor concentrations, increasing from left to right, and the y-axis represents the fold change in peptide abundance upon drug treatment. A fold change of 1 indicates no abundance change. The annotated values indicate the half-maximal inhibitory concentrations of the three inhibitors for HSP90AA1 obtained from PELSA calculations. (C) Affinity characterization of the three heat shock protein inhibitors with purified HSP90AA1 using microscale thermophoresis (MST). The annotated values represent the binding constants between HSP90AA1 and the three heat shock protein inhibitors determined by MST.

### EXAMPLES

To provide a clearer explanation of the technical solutions and highlight the advantages of the present invention, the following detailed description is presented in conjunction with specific examples. It should be noted that these examples are not intended to limit the scope of the invention.

In the following examples: Example 1 and Examples 4-9 illustrate the capability of the method to identify proteins and protein regions whose energy states are altered upon binding with anticancer drugs, metabolites, antibodies, peptides with post-translational modifications, or metal ions. Example 2 validates that PELSA surpasses the existing method, LiP-MS, in identifying a greater number of peptides responsive to changes in energy states or ligand binding. Furthermore, these peptides demonstrate larger fold change amplitudes in PELSA. Example 3 establishes that PELSA is currently the most sensitive method available for the identification of proteins and protein regions with altered energy states. Example 10 illustrates the utilization of non-specific or specific proteases (except trypsin) in PELSA also enable the identification of proteins undergoing energy state alterations. Example 11 demonstrates the application of a dimethyl labeling-based quantitative approach for peptide quantification in PELSA. Example 12 exemplifies the determination of the affinity between ligands and their binding regions using PELSA. Additionally, Examples 1-9 showcase the versatility of the method in identifying ligand-binding proteins and their corresponding binding regions for drugs, broad-spectrum kinase inhibitors, metabolites, antibodies, peptides with post-translational modifications, and metal ions. Additionally, the demonstrated binding of ligands to target proteins in Examples 1-9 can induce conformational changes in the target proteins, thereby highlighting the method's applicability in studying protein conformational alterations.

In Example 1, treating BT474 cell lysate with 100 nM lapatinib led to remarkable energy state alterations (-log10Pvalue > 5) specifically in the known target protein of lapatinib, ERBB2. The PELSA-detected energy state change precisely occurred in the region corresponding to the kinase domain, confirming that PELSA is capable of identifying proteins and regions with altered energy states, as well as the binding proteins and binding regions of ligands.

Furthermore, based on the PELSA results, it was observed that when the lapatinib concentration was increased to 1 µM, a higher number of off-target kinase proteins were identified.

In Example 2, a comparison between LiP-MS and PELSA was conducted for the identification of proteins with energy state changes induced by MTX and SHP099 binding in HeLa cell lysate. The results demonstrated that PELSA was able to identify a greater number of peptides responsive to changes in protein energy states or ligand binding compared to LiP-MS (2-5 times more). Additionally, these peptides exhibited larger amplitudes of fold changes in response to ligand binding or energy state alterations compared to LiP-MS (4-6 times).

Example 3 involved the identification of staurosporine kinase target proteins using PELSA in K562 and HeLa cell lysates. PELSA successfully identified 121 and 111 kinase target proteins in K562 and HeLa cell lysates, respectively. These numbers were 2.1 times higher than the reported TPP method (53) and 12.3 times higher than the number of kinase target proteins identified by the reported LiP-Quant method (9). This result highlights that PELSA is the most sensitive for identifying proteins with altered energy states.

Examples 4-6 demonstrated the high sensitivity of PELSA in detecting weak protein-ligand interactions and accurately locating the binding regions of the ligands.

In Example 7, PELSA successfully identified the epitopes of antibodies in HeLa cell lysate, indicating its capability to identify protein-protein interaction interfaces.

In Example 8, PELSA successfully identified the recognition domains for tyrosine phosphorylation (pYEEI) in BT474 cell lysates, providing a powerful tool for identifying recognition domains involved in other post-translational modifications.

In Example 9, PELSA successfully identified 112 Zn2+-binding proteins and accurately located the Zn2+-binding regions in cell lysates. This finding demonstrates that PELSA can identify ligand-protein interactions regardless of ligand size. Moreover, based on the PELSA results, the addition of zinc ions stabilized the calcium ion binding motif (EF-hand/EH motif) while destabilizing the IQ motif, which interacts with the EF-hand motif. This suggests that zinc ion binding to the EF-hand motif leads to dissociation from the IQ motif, resulting in the destabilization of the IQ motif. These results also emphasize the potential of PELSA in studying binding interfaces of protein complexes.

Example 10 investigated the use of various proteases in PELSA other than trypsin. Although the identification of peptides and proteins using chymotrypsin and proteinase K was lower compared to trypsin, they still enabled the identification of MTX-binding proteins. This suggests that the utilization of proteases other than trypsin in PELSA could enable the identification of binding proteins as well.

In Example 11, the combination of PELSA with dimethyl labeling was explored to identify target proteins of heat shock protein inhibitors. The results showed that by implementing the strategy of selecting peptide with the second-largest absolute value of fold change, PELSA achieves high specificity in identifying both target proteins and highly confident off-target proteins.

Example 12 demonstrates that PELSA provides a high level of confidence in determining the binding affinity between ligands and proteins.

### Example 1

PELSA is employed to identify the proteins and protein regions that undergo changes in energy state after treatment of BT474 cell lysate with the anticancer drug lapatinib.
(1) Take a dish of BT474 cells (approximately 5e7 cells) and resuspend them in 1 mL lysis buffer (PBS supplemented with 1% (v/v) protease inhibitor (Sigma, catalog number P8340-5mL)). Freeze-thaw the mixture three times (freeze in liquid nitrogen for two minutes, thaw in a 37°C water bath for two minutes, repeat three times) to obtain crude cell lysates. Centrifuge the crude cell lysates at 500 g, 4°C for 10 minutes, and collect the supernatant as the cell lysate. Determine the protein concentration using the PierceTM 660 nm Protein Assay (Thermo, USA).
(2) Adjust the protein concentration of the cell lysate to 1 mg/mL using cell lysis buffer. Take four portions of the cell lysate in EP tubes, each containing 50 µL. Add 0.5 µL of lapatinib (Selleck, catalog number S2111) at different concentrations to achieve final concentrations of 100 nM, 1 µM, 10 µM, and 100 µM (lapatinib stock concentrations are 10 µM, 100 µM, 1 mM, and 10 mM respectively; lapatinib dissolved in DMSO) as experimental groups. Take another 50 µL of cell lysate and add 0.5 µL of DMSO as the control group. Perform four replicate experiments for both the experimental and control groups and incubate the cell lysates with drug or vehicle at room temperature for 30 minutes.
(3) Add trypsin (Sigma, catalog number T1426) to the experimental and control groups at a ratio of 1:2 (weight/weight, wt/wt) of protease to protein. Digest the samples on a shaker at 37°C, 1000 rpm for 1 minute. Add 165 µL of pH 8.2 HEPES (Sigma, catalog number H3375) buffer containing 8 M guanidine hydrochloride (Sigma, catalog number G3272) to stop the digestion.
(4) Add TCEP (Sigma, catalog number C4706) to a final concentration of 10 mM and CAA (Sigma, catalog number 22790) to a final concentration of 40 mM to the digested sample. Heat the sample at 95°C for 5 minutes, cool it to room temperature, and then transfer the sample to a 10 kDa cutoff ultrafiltration tube (Sartorius, catalog number VN01H02). Centrifuge at 14,000 g for 50 minutes and collect the filtrates. Wash the membrane with 200 µL of pH 8.2 HEPES buffer and collect the filtrates. Combine the filtrates obtained from two rounds of ultrafiltration.
(5) Desalt the filtrates i.e., peptides, using a 200 µL tip filled with 2 mg of HLB particles (Waters, USA). The desalted peptides are dried using Speed-vac system (Thermo, USA)
(6) The above-mentioned peptide mixture was reconstituted in 20 µL of 0.1% (v/v) formic acid (Sigma, Catalog No. V900803) and subjected to LC-MS/MS analysis. Each sample was analyzed once using data-independent acquisition (DIA) mass spectrometry. The Spectronaut software was used to identify and quantify the peptides, providing information on the corresponding protein, the peptide's location within the protein, and its abundance in each sample. In this implementation, we first analyzed the secondary structure elements of PELSA proteolytic sites. The results showed that 59% of the cleavage sites in PELSA experiments were located within the protein's helical structure (FIG. 2A), suggesting that PELSA disrupted the protein structure, resulting in the digestion of protein regions, even in stable, low-energy states.
(7) The abundance of peptides in the lapatinib-treated and untreated groups was statistically evaluated using Empirical Bayes t-test to determine the P-value and fold change for each peptide, representing the significance and magnitude of the peptide's abundance change upon lapatinib treatment. For each protein, the peptide with the lowest P-value (highest significance) among all its peptides was chosen to represent the protein. In this study, proteins with a - log10Pvalue > 5 were considered to undergo changes in energy state. In FIG. 2B, among the 5,774 quantified proteins, only the known target protein of lapatinib, ERBB2, exhibited significant fold changes under treatment with 100 nM lapatinib.

ERBB2 is a receptor tyrosine kinase located on the cell membrane. It consists of extracellular, transmembrane, and intracellular regions, including the kinase domain and non-kinase domain. Lapatinib specifically targets the kinase domain of ERBB2. In FIG. 2C, x-axis denotes the protein sequence from the N-terminus to the C-terminus with square representing the kinase domain, and the y-axis represents the fold change in abundance of ERBB2 peptides under 100 nM lapatinib treatment. Significantly altered peptides (|log2(fold change)| > 0.3 & -log10(P value) > 2) were found only within or in close proximity to the kinase domain. This result demonstrates the PELSA's ability to determine the drug-binding region.

Furthermore, it was observed that at higher concentrations of lapatinib (100 µM, 10 µM, and 1 µM), the peptides within the kinase domain of ERBB2 were more resistant to proteolysis (FIG. 2D). Additionally, it was noted that while only ERBB2 exhibited significant changes in energy state at a lapatinib concentration of 100 nM, increasing the lapatinib concentration led to energy state changes in several other kinases such as CHEK2, SLK, RIPK2, and YES1 (FIG. 2E). Most of the altered peptides in these kinases were also located within their respective kinase domains (FIG. 2F). These findings indicate that lapatinib, at higher doses, interacts with other kinases, suggesting that our method can be used to assess drug promiscuity and guide drug rational design.

Furthermore, we also observed that the non-kinase protein PTGES2 exhibited changes in its energy state at high concentrations of lapatinib (FIG. 2E). To validate the binding of PTGES2 and lapatinib, we performed Western blotting using a similar procedure and conditions as mentioned above, with the following modifications: The lysis buffer is PBS. After lysis, the resulting cell lysates were divided into 8 portions and treated with equal volumes of lapatinib at different final concentrations (lapatinib dissolved in DMSO), as well as DMSO, resulting in final lapatinib concentrations of 100 µM, 50 µM, 10 µM, 1 µM, 0.1 µM, 0.01 µM, 0.001 µM, and 0. The mixtures were incubated at room temperature (25°C) for 30 minutes. After incubation, trypsin was added at a 1:40 protease-to-protein weight ratio, and the mixture was subjected to digestion at 37°C for 1 minute. The digestion was terminated by heating the samples to 95°C, followed by the addition of 1/4 volume of 5*loading buffer, and heating at 95°C for another 5 minutes. Western blotting was employed to detect the undigested proteins. The gel was concentrated at 80 V for 20 minutes, and then separated at 120 V for 60 minutes. Transfer blotting was conducted at a constant current of 250 mA for 40 minutes. After blocking, the primary antibody against PTGES2 (Proteintech, USA) was added at a 1:800 dilution and incubated at room temperature for 1 hour. This was followed by incubation with a goat anti-rabbit HRP-IgG secondary antibody (Abcam, UK) at room temperature for 1 hour. Chemiluminescent detection was performed using ECL reagent (Thermo Fisher Scientific, America) and the Fusion FX5 chemiluminescence system (Vilber Infinit, France). The undigested protein bands were quantified based on their intensities, using GAPDH as a reference protein. For this, the membrane was incubated with a rabbit anti-mouse HRP-IgG secondary antibody (Abcam, UK) at room temperature for 1 hour, and chemiluminescent detection was performed. As shown in FIG. 2G, with increasing concentrations of lapatinib, the bands became progressively darker, indicating an increasing amount of PTGES2 protein protected from digestion. The maximum value was reached at a lapatinib concentration of 50 µM. These results suggest that the susceptibility of PTGES2 to proteolysis depends on the lapatinib concentration and thus provides further evidence supporting the notion that PTGES2 may be an off-target protein of lapatinib.

The above analysis results demonstrate that PELSA can identify ligand-binding proteins with high specificity and determine the ligand-binding regions based on the peptides with changed abundance. This unbiased nature of PELSA also facilitates identifying off-target proteins, such as other kinases and PTGES2, as observed in this study. This indicates that the method can evaluate drug promiscuity and provide guidance for rational drug design. Furthermore, only peptides within the ligand-binding regions showed significant abundance fold changes, indicating that the method can reveal the drug's binding regions. The dose-dependent fold changes in peptide abundance suggest the capacity of PELSA to determine the binding affinities between ligand and the target proteins.

### Example 2

Comparison between LiP-MS and PELSA for the identification of proteins with changes in energy state upon treatment with methotrexate (MTX) and SHP099 in HeLa cell lysates.

To showcase the advantages of PELSA in identifying proteins with changes in energy state, this example compares the existing method LiP-MS with the present invention (PELSA) for identifying proteins exhibiting energy state changes after MTX and SHP099 treatment in HeLa cell lysates. DHFR is the target protein of MTX, and PTPN11 is the target protein of SHP099. In this example, the PELSA procedure and experimental conditions are the same as in Example 1, except for the following differences: HeLa cell samples are used, and the ligands are MTX (Selleck, catalog number S1210) at a final concentration of 10 µM or SHP099 (Selleck, catalog number S8278) at a final concentration of 10 µM. The LiP-MS procedure adheres to the protocol described in the literature by Piazza et al. (Nature Communication, 2020, 11(1):4200), with the following steps (The following describes the procedures and experimental conditions that differ from those in Example 1):
(1) The cell lysis buffer used is composed of 60 mM HEPES pH 7.5, 150 mM KCl, and 1 mM MgCl2 (Piazza et al., Nature Communication, 2020, 11(1):4200).
(2) After incubation of the cell lysate with the drug, Proteinase K (Sigma, catalog number P2308) is added at a 1:100 (wt/wt) ratio of protease to protein. The mixture is incubated at 25°C, 1000 rpm on a shaker for 4 minutes, heated at 98°C for 1 minute, and then an equal volume of 10% sodium deoxycholate (Sigma, catalog number D6750) is added. The mixture is heated at 98°C for another 4 minutes to denature the protein fragments.
(3) After denaturation, final concentrations of 10 mM TCEP (Sigma, catalog number C4706) and 40 mM CAA (Sigma, catalog number 22790) are added. The sample is heated at 98°C for 5 minutes, cooled to room temperature, and subsequently diluted with a four-fold volume of pH 8.2 60 mM HEPES buffer to attain a final sodium deoxycholate concentration of 1%.
(4) Lys-C (Wako chemicals) is added at a 1:100 (wt/wt) ratio of protease to protein and incubated for 4 hours. Subsequently, trypsin (Promega) is added at a 1:50 (wt/wt) ratio of protease to protein and incubated for 16 hours.
(5) After the digestion, formic acid (FA) is added to achieve a final volume of 1.5%. The sample is left to settle for 10 minutes, and once the sodium deoxycholate precipitate has achieved equilibrium, it is centrifuged at room temperature for 10 minutes at 20,000 g (twice) to remove the sodium deoxycholate precipitates.
(6) The peptide desalting, mass spectrometry quantification, software searching, and data analysis processes are the same as in Example 1, except when searching with Spectronaut, Enzyme was set as "trypsin" and digest type was set as "semi-tryptic".

In FIG. 3A and 3B, the peptides that meet the criteria of -log10Pvalue > 2 and |log2 fold change| > 0.3 are defined as ligand-responsive peptides. In the MTX-DHFR system, PELSA identified 2 times more MTX-responsive DHFR peptides than LiP-MS. Similarly, in the SHP099-PTPN11 system, PELSA identified 5.25 times more SHP099-responsive PTPN11 peptides than LiP-MS. Furthermore, the response magnitude of DHFR peptides to MTX in PELSA was 4.3 times higher than in LiP-MS (FIG. 3C), and the response magnitude of PTPN11 peptides to SHP099 in PELSA was 6.4 times higher than in LiP-MS (FIG. 3C). For example, in the MTX-DHFR system, a peptide located at the MTX-binding site displayed significant fold changes in PELSA experiments, whereas no fold change was detected in the same peptide in LiP-MS experiments (FIG. 3D). This can be explained: this peptide adopts a helical structure in a low-energy state, and the brief digestion in LiP-MS may not reach this structure. In contrast, PELSA's disruptive digestion enables the detection of changes in regions with lower protein energy states, therefore allowing for the detection of ligand binding with this peptide. Likewise, in the SHP099-PTPN11 system, a peptide positioned at the SHP099-binding site and embedded within the protein structure exhibited fold changes exclusively in PELSA experiments (FIG. 3E). This finding further underscores PELSA's capability to disrupt internal, stable protein structures, thereby capturing alterations in the protein energy state that occur within these regions. These findings suggest that PELSA surpasses LiP-MS in identifying a larger number of ligand-responsive target protein peptides. Moreover, the ligand-responsive peptides identified by PELSA exhibit significantly higher response magnitudes compared to LiP-MS. This heightened sensitivity of PELSA enables the detection of GART, a protein involved in MTX pharmacokinetics, which shows energy state changes exclusively through PELSA (FIG. 3A). The implementation demonstrates that the present invention (PELSA), compared to the existing LiP-MS method, can generate more ligand-responsive peptides and these ligand-responsive peptides display large magnitudes of fold changes in response to ligand binding. Consequently, it enables the identification of a larger number of ligand-binding proteins. This partly explains the exceptionally high sensitivity of the present invention in ligand-binding protein identification.

### Example 3

PELSA identification of the proteins and protein regions in HeLa and K562 cell lysates that undergo changes in energy state upon treatment with a pan-kinase inhibitor staurosporine.

This implementation demonstrated the high sensitivity of PELSA in identifying proteins undergoing energy state changes, as supported by its identification of a large number of staurosporine-binding proteins and comparisons with the performance of the thermal proteome profiling (TPP) method and LiP-Quant, as reported in the literature.

The experimental procedure and conditions were similar to Example 1, with the exception of using K562 and HeLa cell samples. The experimental group was treated with 20 µM staurosporine (final concentration, Selleck, catalog number S1421). As depicted in FIG. 4A, PELSA revealed that a large number of kinases were stabilized in both HeLa and K562 lysates upon staurosporine treatment. By setting cutoff of kinase proportion among the identified target proteins to 80%, PELSA identified 121 kinase targets (out of a total of 143 target proteins) in K562 cell lysate and 111 kinase targets (out of a total of 135 target proteins) in HeLa cell lysate. In contrast, according to published literature, LiP-Quant only identified 9 kinase targets for staurosporine using the same cutoff of kinase proportion (i.e., ≥80%), while TPP identified 53 kinase targets for staurosporine (out of a total of 60 target proteins) (FIG. 4B). These findings unequivocally demonstrate the exceptional sensitivity of PELSA compared to existing methods. We further compared the protein and peptide coverage depths of LiP-Quant, TPP, and PELSA. We observed that although in LiP-Quant experiment, a greater number of peptides were identified (FIG. 4C), and LiP-Quant achieved higher overall protein sequence coverages compared to PELSA (FIG. 4D), PELSA identified a significantly larger number of kinase targets than LiP-Quant (111 versus 9). Further analysis revealed that the sequence coverages of kinase targets identified by PELSA were lower than those identified by LiP-Quant in LiP-Quant experiment (FIG. 4D). This suggests that PELSA is capable of identifying target proteins even with lower sequence coverages. In contrast, LiP-Quant, which includes many irrelevant peptides resulting from complete digestion, requires higher protein sequence coverages to identify target proteins.

TPP is a method that can only output changes in energy state at the protein level. Although TPP identified more proteins (7673) compared to PELSA-K562 (6310) (FIG. 4C), PELSA ultimately identified 2.28 times more kinase targets than TPP, further highlighting the high sensitivity of PELSA in target protein identification. Analysis of the melting points of kinase targets identified by TPP and PELSA revealed that TPP has a limited capacity in identifying kinase targets with very high or very low melting temperatures. In contrast, PELSA effectively identifies kinase targets across a wide range of melting temperature points (FIG. 4E). PELSA identified a total of 192 staurosporine target proteins in the two cell lines, of which 154 were kinases, accounting for 80% of all target proteins (FIG. 4F). Additionally, PELSA is capable of identifying the binding regions of staurosporine, as shown in FIG. 4G. Within the significantly changed peptides identified by PELSA, over 92% of the peptides were located within or in close proximity (within 10 amino acid residues) to kinase domains in both K562 and HeLa lysates.

The experimental results indicate that PELSA not only exhibits extremely high sensitivity in the identification of ligand-binding proteins but also accurately identifies the binding regions of ligands on proteins.

### Example 4

PELSA identification of proteins and protein regions undergoing changes in energy state upon treatment with the metabolite folate in K562 cell lysate.

Unlike the strong interaction between lapatinib and ERBB2 (with an affinity of approximately 9 nM), folate exhibits weaker binding affinity to its binding proteins. For instance, previous research indicates that folate binds to its target protein DHFR with Ka values ranging from 3 to 60 µM (Ozaki Y et al., Biochemistry, 1981, 20(11): 3219-3225). Therefore, we employed folate to investigate whether PELSA is capable of analyzing the changes in protein energy state induced by low-affinity ligand binding.

Most experimental procedures and conditions were the same to Example 1, except the followings: K562 cell samples were used. After subjecting the cell samples to three rounds of freeze-thaw cycles (freeze with liquid nitrogen and thaw in a water bath), the supernatant was obtained by centrifugation at 500 g, 4°C for 10 minutes. To remove endogenous folate, a protein desalting step was performed using Zeba Spin desalting columns (Thermo Fisher Scientific). The protein concentration of the desalted lysates was determined using the PierceTM 660nm Protein Assay (Thermo, USA). The protein concentration was adjusted to 1 mg/mL using cell lysis buffer. The ligand used in this experiment was folate (Sigma, catalog number F7879) at a final concentration of 50 µM. The subsequent steps were carried out as described in Example 1.

As shown in FIG. 5A, the stability of the known folate target protein DHFR exhibited the most significant changes. By mapping the DHFR peptides onto its protein structure (FIG. 5B), we observed that the regions experiencing the most notable stability changes corresponded to the binding site of folate. Furthermore, we identified stability changes in three proteins (ATIC, MTHFR, and GART) known to interact with folate analogs. Interestingly, the stabilized regions of these proteins coincided with the binding sites of folate analogs (FIG. 5C-5E). P3H1, is a prolyl 3-hydroxylase involved in collagen prolyl hydroxylation, and literature suggested that folate can participate in proline hydroxylation in collagen (Haustvast J, et al., British Journal of Nutrition, 1974, 32(2): 457-469). Our experimental results revealed that the hydroxylase domain of P3H1 was stabilized upon the addition of folate (FIG. 5F), providing valuable evidence for folate's involvement in collagen prolyl hydroxylation.

### Example 5

PELSA identification of proteins and protein regions undergoing changes in energy state upon treatment with the metabolite leucine in K562 cell lysate.

Leucine has low affinities with its target proteins, LARS1 and SESN2; the reported dissociation constants are 95 µM (Kim S, et al., Cell Reports, 2021, 35(4): 109301) and 20 µM (Wolfson RL, et al., Science, 2016, 351(6268): 43-48), respectively. Therefore, we also used leucine to assess the applicability of PELSA in analyzing the changes in protein energy state induced by weak ligand-protein interactions.

The procedures and conditions followed those outlined in Example 4. Except that leucine (Sigma, catalog number 61819) was used as the investigated ligand with a final concentration of 5 mM. As depicted in FIG. 5G, we observed significant changes in the energy states of well-known leucine target proteins, including LARS1, LARS2, GLUD1, and SENS2. LARS1 possesses two leucine-binding sites located in the CD and CP domains, corresponding to the synthetic site and editing site, respectively. We noted that the peptides from the synthetic site (labeled as CP in the FIG. 5H) exhibited more pronounced fold changes compared to those from the editing site (labeled as CD in the FIG. 5H). Moreover, peptides from the C-terminal domain, which does not contribute to leucine binding, showed no response (FIG. 5H). These findings indicate the ability of PELSA to differentiate between distinct binding sites on a single protein. We also observed significant changes in the energy state of SLC1A5, another known leucine-binding protein (FIG. 5G). SLC1A5 is a membrane protein comprising intracellular, transmembrane, and extracellular regions. The Na-dicarboxylate_symporter domain (residues 54-483), located in the extracellular region, plays a key role in amino acid transport. Among the identified peptides of SLC1A5, only the peptide (residues 190-212) from Na-dicarboxylate_symporter domain showed a substantial change (FIG. 5I), demonstrating the capability of our method to identify membrane protein targets and determine the binding regions. Interestingly, we also discovered that leucine stabilized PPIP5K1 and PPIP5K2, which were not previously known to bind leucine (FIG. 5G), specifically at their shared histidine phosphatase domains (FIG. 5J).

This example showcases the ability of PELSA to detect weak interactions between metabolites and proteins and to accurately identify the binding regions of the metabolites. Additionally, the successful identification of SLC1A5 as a leucine target protein demonstrates the efficacy of PELSA in identifying membrane protein targets. PELSA also identified several novel leucine-binding proteins, shedding light on future investigations into the functions of leucine.

### Example 6

PELSA identification of proteins and protein regions undergoing changes in energy state upon treatment with the metabolite alpha-ketoglutarate (αKG) in HeLa cell lysate.

The procedures and conditions followed those outlined in Example 4. Except that the cell lysate was derived from HeLa cells and αKG (Sigma, catalog number 75890-25g) was used as the investigated ligand with a final concentration of 2 mM. Proteins that met the criteria of - log10Pvalue > 3.4 and log2FC < -0.5 were considered stabilized by 2 mM αKG.

As depicted in FIG. 5K, PELSA identified 40 proteins that were stabilized by 2 mM αKG in HeLa cell lysate, out of which 30 were already known αKG target proteins. This represents the highest number of known αKG target proteins identified in a single experiment. While literature reports have used LiP-MS to identify αKG target proteins in Escherichia coli (Piazza I et al., Cell, 2018, 172(1-15)), only 2 out of the 34 identified target proteins were previously known αKG-binding proteins. Additionally, two-dimensional local stability profiles (FIG. 5L) demonstrated that the protein regions undergoing energy change precisely matched the previously-known αKG-binding regions.

In summary, this example emphasizes PELSA's exceptional sensitivity in analyzing weak interactions between metabolites and proteins, along with its capacity to pinpoint the binding regions of metabolites.

### Example 7

PELSA identification of proteins and protein regions undergoing changes in energy state upon treatment with antibodies in HeLa cell lysate.

This example demonstrates the application of PELSA in identifying antibody-binding epitopes by identifying protein regions that undergo changes in energy state after antibody treatment of HeLa cell lysate.

Most experimental procedures and conditions were the same to Example 1, except the followings: The cell lysate was from HeLa cells and two commercial antibodies, DHFR antibody (Wabways, China, RRID: AB_2877179) and CDK9 antibody (Wabways, China, RRID: AB_2877178), were used as the investigated ligands with a final concentration of 2% (v/v) and 1% (v/v), respectively. Proteins exhibiting a -log10P value > 5 were considered to undergo changes in energy state. FIG. 6A illustrates the schematic representation of identifying antibody-binding epitopes in cell lysate. FIG. 6B (left) depicts the volcano plot of proteins obtained from DHFR antibody-treated HeLa cell lysate, whereas FIG. 6B (right) shows the volcano plot of proteins obtained from CDK9 antibody-treated HeLa cell lysate. The addition of DHFR and CDK9 antibodies caused significant changes in the energy states of DHFR and CDK9, respectively, indicating the capability of PELSA to directly identify antigen proteins in cell lysate. Notably, other proteins showing changes in energy state might be attributed to non-specific binding with the antibody.

PELSA also revealed that the peptides detected to undergo changes in energy state precisely corresponded to the known epitopes recognized by the antibody (FIG. 6C and 6D). For CDK9, two peptides exhibited abundance changes in opposite directions. The CDK9 antibody recognized the epitope sequence PATTNQTEFERVF located at the C-terminus of CDK9. The sequence NPATTNQTEFER contained a residue within the epitope, resulting in a reduced peptide yield upon antibody addition (FIG. 6D). Conversely, the sequence NPATTNQTEFERVF (NPxxVF), which fully encompassed the epitope sequence but had no the trypsin cleavage site located in the epitope, exhibited an increased peptide yield upon antibody addition (FIG. 6D). Further analysis revealed that the summed intensities of these two peptides were minimally affected by the treatment of the antibody (FIG. 6E). Thus, the opposite changes observed in these two peptides can be attributed to the protective effect of the antibody, rendering the C-terminal R site of the NPATTNQTEFER sequence less susceptible to cleavage, resulting in a decreased yield of the corresponding peptide. As consequence, the NPATTNQTEFERVF (NPxxVF) sequence, which should remain unchanged by CDK9 antibody treatment, were left more. These results demonstrate the high resolution of PELSA in identifying antibody-binding epitopes.

This example highlights the ability of PELSA to accurately identify interactions between antibodies and proteins, revealing the binding sites of antibodies. Furthermore, it indicates the potential of PELSA to be applied to various protein-protein interaction systems and beyond.

### Example 8

PELSA identification of proteins and protein regions undergoing changes in energy state upon treatment with post-translational modified peptides in BT474 cell lysate.

Post-translational modifications (PTMs) of proteins play a crucial role in various biological activities. The regulation of these activities often requires the recognition and recruitment of effector proteins by downstream proteins. Therefore, the identification of proteins that recognize PTMs is essential for understanding functions of PTMs and studying disease mechanisms mediated by PTMs. Phosphorylated tyrosine-glutamate-glutamate-isoleucine (referred to as pYEEI) is known to be recognized by proteins containing SH2 domains. In this analysis, we will utilize PELSA to identify the protein regions involved in recognizing PTMs by studying proteins and protein regions that undergo changes in energy state upon treatment with pYEEI in BT474 cell lysates.

Most experimental procedures and conditions were the same to Example 1, except the followings: To prevent the phosphorylated peptide from being dephosphorylated by active phosphatases present in the cell lysate, an additional 2 mM phosphatase inhibitor is added to the cell lysis buffer. For parallel comparison with results of pulldown experiments, we utilized a biotinylated form of the phosphorylated peptide, specifically N-terminal biotinylated pYEEI (abbreviated as Biotin-pYEEI, synthesized by Qiangyao Biotechnology). The concentration used for the ligand addition is 100 µM. To eliminate any potential influences arising from the phosphate group and YEEI, two control groups were included. One control group was treated 100 µM N-terminal biotinylated phosphorylated serine-glutamate-glutamate-isoleucine (abbreviated as Biotin-pSEEI, synthesized by Qiangyao Biotechnology), while the other control group was treated with 100 µM N-terminal biotinylated tyrosine-glutamate-glutamate-isoleucine (abbreviated as Biotin-YEEI, synthesized by Qiangyao Biotechnology).

The pulldown experiment was conducted as follows: The cell lysate is from BT474 cells, and the cell lysis was performed as described in Example 1. After adjusting the protein concentration of the cell lysate to 1 mg/mL, 100 µL of the cell lysate was taken and treated with Biotin-pYEEI at a final concentration of 100 µM as the experimental group. Two additional 100 µL samples were treated with Biotin-pSEEI at a final concentration of 100 µM and Biotin-YEEI at a final concentration of 100 µM, respectively, serving as control groups. This process was repeated three times, and the samples were incubated with ligands at room temperature for 30 minutes. After incubation, 200 µL of avidin beads (Thermo, USA) were added to each group and incubated overnight at 4°C. Subsequently, the beads were washed four times with a washing buffer (1% phosphatase inhibitor, and 0.5% NP40 in PBS), followed by four washes with a cell lysis buffer (1% phosphatase inhibitor in PBS solution). The proteins were eluted by adding 100 µL of HEPES buffer (pH 8.2) containing 8M guanidine hydrochloride, and the elution step was repeated twice. The eluted proteins were alkylated by adding 10 mM TCEP and 40 mM CAA, followed by heating at 95°C for 5 minutes. The eluted protein solution was transferred to an ultrafiltration unit and centrifuged at 14,000 g for 30 minutes to remove the buffer. The ultrafiltration membrane was washed twice with 200 µL of a 10 mM ammonium bicarbonate (NH4HCO3) buffer. Then, 100 µL of a 10 mM NH4HCO3 buffer was added to resuspend the proteins retained on the membrane, and 2 µg of trypsin (Promega) was added for overnight digestion. The next day, the ultrafiltration tube was centrifuged at 14,000 g for 50 minutes to collect the peptides. The ultrafiltration membrane was washed with 100 µL of NH4HCO3 buffer and centrifuged at 14,000 g for another 50 minutes to collect the remaining peptides. The peptides collected from two cycles of centrifugation were pooled and subjected to freeze-drying. The procedures of mass spectrometry analysis, software analysis, and data validation were the same as described in Example 1.

FIG. 7A depicts a schematic diagram illustrating the identification of tyrosine phosphorylation recognition domains in cell lysates. Using YEEI as the control group, the Empirical Bayes t-test yielded the Pvalue (pYEEI/YEEI). Using pSEEI as the control group, the Empirical Bayes t-test yielded the Pvalue (pYEEI/pSEEI). The logarithm of both P-values was plotted, and proteins meeting the criteria of -log10Pvalue (pYEEI/YEEI) > 3.1, log2FC (pYEEI/YEEI) < 0, -log 10Pvalue (pYEEI/pSEEI) > 3.1, and log2FC (pYEEI/pSEEI) < 0 were defined as proteins with a reduced energy state upon treatment of pYEEI, i.e., stabilized by treatment of pYEEI. This results in the identification of 28 proteins. Among these 28 proteins, 9 contained the SH2 domain, and an additional 8 proteins were found to be associated with Ca2+ binding (FIG. 7B). However, in the pulldown experiment, none of the proteins containing the SH2 domain was determined as pYEEI-binding proteins (FIG. 7C), possibly resulting from the strong washing conditions used in pulldown experiment. PELSA also facilitated the identification of the recognition domains for pYEEI: as shown in FIG. 7D and 7E, only peptides within the SH2 domain were stabilized upon the addition of pYEEI, while the abundance of other peptides remained unchanged. Furthermore, the identified Ca2+-related proteins were only stabilized in EF-hand domains, the known Ca2+-binding domains (FIG. 7F).

These results demonstrate that PELSA can identify proteins and protein regions that undergo changes in energy state induced by binding of post-translationally modified peptides.

### Example 9

PELSA identification of proteins and protein regions undergoing changes in energy state upon treatment with metal ions in HeLa cell lysate.

In this example, we investigated whether PELSA can be applied to detect changes in protein energy state induced by binding of small-sized metal ions.

Most experimental procedures and conditions were the same to Example 1, except the followings: the cell lysate was from HeLa cells, and the cell lysis buffer was supplemented with 2 mM ethylenediaminetetraacetic acid sodium salt (EDTA, purchased from Sigma) to chelate the metal ions present in the cell lysate. After cell lysis following the procedure described in Example 1, the added EDTA was removed using Zeba Spin desalting columns (Thermo Fisher Scientific) through two rounds of protein desalting. A final concentration of 30 µM zinc chloride (Sigma, catalog number 450111-10G) was added to the 50 µL cell lysate, serving as the experimental group. Proteins meeting the criteria of -log10Pvalue > 3 and log2FC < -0.5 were considered as proteins stabilized by Zn2+.

FIG. 8A depicts that PELSA identified a significant number of metal ion-binding proteins that were stabilized by treatment of Zn2+: among all 280 stabilized by treatment of Zn2+, more than 66% (185 proteins) were already known metal ion-binding proteins. In contrast, within the entire identified proteome, the proportion of metal ion-binding proteins was only 19%, indicating that PELSA can successfully identify metal ion-binding proteins by detecting changes in energy state upon Zn2+ treatment. Zn2+ has been reported to bind to the EF-hand motifs of Ca2+-binding proteins (Tsvetkov P O et al., Front Mol Neurosci, 2018, 11: 459) and occupy Mg2+-binding sites in Mg2+-binding proteins (Dudev T et al., Chemical Reviews, 2003, 103(3): 773-787), suggesting the promiscuous nature of these divalent metal ions in metal ion-binding proteins. Consistent with the literature, we found 73 proteins that were stabilized by zinc ions were known binding proteins of other metal ions (FIG. 8B). Among these 78 proteins, 25 were known Ca2+-binding proteins, 20 were known Mg2+-binding proteins, 15 were known Fe2+-binding proteins, 9 were known Mn2+-binding proteins, and 6 were known binding proteins of other metal ions (FIG. 8C). Within the group of 112 Zn2+-binding proteins, 60 proteins contained zinc finger motifs (FIG. 8C). Analyzing the proteins with zinc finger motifs revealed that the median absolute value of log2FC for peptides within the zinc finger motifs was significantly larger compared to peptides outside the zinc finger motifs (FIG. 8D and 8E). These results indicate the ability of PELSA to accurately identify Zn2+-binding sites. Furthermore, in the case of proteins lacking zinc finger motifs, such as LIMA1 which possesses a LIM Zn2+-binding domain, three peptides from this domain were quantified using PELSA (FIG. 8F). Among these peptides, the one directly involved in Zn2+ binding exhibited the largest fold change, while the other two peptides not directly involved in Zn2+ binding showed minimal changes (|log2FC| < 0.3, FIG. 8G). These results strongly support the high precision of PELSA in accurately localizing metal ion binding sites.

Analysis of the identified Ca2+-binding proteins revealed that out of the 27 proteins, 20 of them contained EF-hand/EH motifs (Ca2+-binding motifs). Similar to proteins that contains zinc finger motifs, the median of absolute log2FC values for peptides within the EF-hand/EH motifs were significantly larger compared to peptides outside these motifs (FIG. 8H), indicating that Zn2+ can bind the EF-hand/EH motifs in these Ca2+-binding proteins. Furthermore, among the 9 proteins containing Fe2+-binding domains, the median of absolute log2FC values for peptides within the Fe2+-binding domains was significantly larger compared to peptides outside these domains (FIG. 8I), indicating that Zn2+ can bind the Fe2+-binding proteins in these Fe2+-binding proteins.

When a ligand binds to a protein, it can dissociate the protein from its original complex, leading to destabilization of the protein's binding partners. We observed destabilization in several proteins containing IQ motifs upon treatment of Zn2+ (-log10Pvalue > 6 and log2FC > 0) (FIG. 8J). Interestingly, the destabilized peptides precisely corresponded to the location of the IQ motifs or were in close proximity to them (FIG. 8K). IQ motifs are known binding sites for EF-hand motifs, which can interact with Zn2+ (FIG. 8H). Therefore, the destabilization of IQ motifs may be attributed to the dissociation between IQ motifs and EF-hand motifs caused by their interaction with Zn2+. IQ motifs and EF-hand motifs are crucial interfaces for protein-protein interactions, suggesting that PELSA can effectively analyze changes in the energy state resulting from the assembly and dissociation of protein complexes and reveal the binding interfaces of proteins within these complexes.

We also observed destabilization in the components of the 26S proteasome regulatory subunits, specifically PSMC1-6 (FIG. 8J). Each of the PSMC1-6 proteins contain a P-Loop-NTPase domain located at the binding interface of the PSMC1-6. PELSA results demonstrated that only the peptides within this domain exhibited significant destabilization (FIG. 8L), indicating that Zn2+ induce the dissociation of the 26S proteasome regulatory subunits, thereby destabilizing the protein-protein interaction interface of PSMC1-6. These findings further underscore the utility of this method in studying the dynamic changes associated with the assembly and dissociation of protein complexes.

### Example 10

Example 10 demonstrates the utilization of non-specific or specific protease (except trypsin) in PELSA to identify proteins undergoing changes in energy state by treatment of HeLa cell lysates with MTX.

To assess the applicability of PELSA in identifying proteins with altered energy states using proteases other than trypsin, we conducted parallel comparisons using trypsin and two other proteases with different cleavage specificities: chymotrypsin (purchased from Sigma, catalog number C3142) and proteinase K (abbreviated as PK, purchased from Sigma, catalog number P2308). These proteases were used to analyze proteins with altered energy states after treating HeLa cell lysate with MTX. Chymotrypsin primarily cleaves at the N-terminus of aromatic amino acids, while proteinase K exhibits broad cleavage specificity.

The procedures and conditions for Trypsin-PELSA are the same to those in Example 1, except the followings: the cell lysate is from HeLa cells; the experimental group was treated with MTX at a final concentration of 10 µM (dissolved in DMSO, with a stock concentration of 1 mM; purchased from Selleck, catalog number S1210).

The procedures and conditions for Chymotrypsin-PELSA are the same to those in Trypsin-PELSA, except the following: trypsin was replaced with chymotrypsin; the digestion conditions were 25°C, 1000 rpm for 1 minute; when searching with Spectronaut, the cleavage sites for the digestion were set as F, W, Y, L, and M.

The procedures and conditions for Proteinase K-PELSA are the same to those in Trypsin-PELSA, except the following: trypsin was replaced with proteinase K; the digestion conditions were 25°C, 1000 rpm for 1 minute; when searching with Spectronaut, the digestion type was set as unspecific.

FIG. 9A shows that Trypsin-PELSA identified a total of 69,245 peptides, while Chymotrypsin-PELSA and Proteinase K-PELSA identified significantly fewer peptides (18,027 and 28,702, respectively) compared to Trypsin-PELSA. Correspondingly, Trypsin-PELSA identified a larger number of proteins (5,487) compared to Chymotrypsin-PELSA (2,710) and Proteinase K-PELSA (1,937) (FIG. 9B). Furthermore, the proteins identified by the Trypsin-PELSA covered the majority of proteins identified by the Proteinase K-PELSA and Chymotrypsin-PELSA (FIG. 9B). This observation can be attributed to the fact that tryptic peptides are more favorable for mass spectrometry identification compared to other types of peptides. In terms of identifying proteins with altered energy states, since DHFR, a known binding protein of MTX, is relatively abundant, all three proteases (trypsin, chymotrypsin, and proteinase K) used in PELSA can detect this protein and distinguished it from the background proteins (FIG. 9C). This example illustrates that PELSA is not limited to the use of trypsin and can also utilize other specific and unspecific proteases to identify proteins with altered energy states. However, it may encounter the issue of a lower number of protein and peptide identifications.

### Example 11

PELSA coupled with dimethyl labeling quantification to identify proteins and protein regions undergoing changes in energy state upon treatment with three heat shock protein 90 (HSP90) inhibitors in HeLa cell lysates.

The procedure is as follows:
(1) The cell lysate was derived from HeLa cells. The process of cell lysis, protein extraction, and protein concentration determination followed the protocol described in in Example 1. Six 50 µL aliquots of cell lysate were prepared in Eppendorf tubes. Three aliquots were treated with 100 µM geldanamycin, 100 µM tanespimycin, and 100 µM ganetespib, respectively (stock concentration: 10 mM; all dissolved in DMSO and purchased from Selleck). The remaining three aliquots were treated with an equal volume of DMSO as the control. The samples were then incubated at room temperature (25 °C) for 30 minutes. After the incubation, trypsin was added to each sample at an protease-to-protein ratio of 1:2 (wt/wt), and the samples were incubated at 37 °C for 1 minute. The digestion was terminated by heating the samples at 100 °C for 5 minutes.
(2) To each sample, 165 µL (i.e., three times volume of the sample) of sodium dihydrogen phosphate buffer (pH 6.5) containing 8 M guanidine hydrochloride was added. The TCEP and CAA were added with final concentrations of 10 mM and 40 mM, respectively. The samples were heated at 95 °C for another 5 minutes for carbamidomethylation and then cooled to room temperature. Subsequently, the samples were transferred to 10 kDa ultrafiltration units and centrifuged at 14,000 g for 50 minutes to isolate the peptides. The ultrafiltration membranes were washed twice with 200 µL sodium dihydrogen phosphate buffer (pH 6.5), and the filtrates from both washes were combined with the initial ultrafiltration filtrate.
(3) Dimethyl labeling: The peptides in drug-treated group were labeled with the medium reagent, i.e., 16 µL of 4% deuterated formaldehyde (Sigma, Cat. No. 596388) and 16 µL of 0.6 M cyanoborohydride (Sigma, Cat. No. 156159). For the control group, 16 µL of 4% formaldehyde (Sigma, Cat. No. 252549) and 16 µL of 0.6 M cyanoborohydride were added as the light labeling. The labeling reaction was carried out at 30 °C for 1 hour. After the reaction, 10 µL of 10% ammonium hydroxide (Sigma, Cat. No. 338818) was added and incubated for an additional 30 minutes. Finally, the peptides from the drug-treated and control groups were mixed in equal amounts for further analysis.
(4) The labeled peptide samples were acidified by adding 4.5 µL of trifluoroacetic acid (Sigma, catalog number T6508). The solution was desalted using a tip column (200 µL capacity) packed with 2 mg of HLB resin (Waters, USA). After desalting, the samples were freeze-dried.
(5) The dried peptides were reconstituted in 30 µL of 0.1% formic acid. Then, 1 µg of peptide was injected and subjected to LC-MS/MS analysis in data-dependent acquisition mode (DDA). Each sample was analyzed twice by mass spectrometer.
(6) The DDA spectral files were analyzed using MaxQuant software (Cox, Germany) to identify the proteins, determine the peptide positions on the proteins, and calculate the fold changes (FC) of peptide abundance between the experimental and control groups.

In this example, three heat shock protein inhibitors with distinct structure similarities were used: geldanamycin, tanespimycin, and ganetespib. Geldanamycin and tanespimycin are HSP90 inhibitors with a benzquinone group, while ganetespib is a structurally distinct second-generation HSP90 inhibitor (FIG. 10A). The second most significantly changed peptide (i.e., the peptide with the second-largest |log2FC| value among all quantified peptides for a given protein) was chosen to represent each protein (FIG. 10B). By considering proteins with |log2FC| > 1.4 as indicators of altered energy states, several HSP90s, including HSP90AB1, HSP90AA1, HSP90A1, and HSP90AB2P, were successfully identified, illustrating the capability of dimethyl labeling-based PELSA to identify proteins with altered energy states. Furthermore, it was observed that the mitochondrial heat shock protein TRAP1 was specifically identified as a binding protein of ganetespib.

HSP90 proteins consist of three domains: the N-terminal ATP-binding domain, the middle domain, and the C-terminal domain. Geldanamycin, tanespimycin, and ganetespib all target the N-terminal ATP-binding domain of HSP90. As shown in FIG. 10C, taking geldanamycin as an example, PELSA analysis revealed significant changes in peptide abundance only in the N-terminal domain, which corresponds to the drug-binding region. This finding further supports the capability of PELSA to accurately identify protein regions with changes in energy state.

In addition to known target proteins, PELSA also identified several off-target proteins. For geldanamycin, PELSA identified destabilization of several PRDX family proteins, with log2FC > 1.4 (FIG. 10B). It has been reported in the literature that geldanamycin can induce the generation of reactive oxygen species (ROS), leading to severe liver toxicity (Clark et al., Free Radical Biology & Medicine, 2009, 1440-1449). PRDX family proteins play a protective role by clearing ROS under oxidative stress conditions. The destabilization of PRDX family proteins suggests that their structures are disrupted at high concentrations of geldanamycin, which provides a possible explanation for the hepatotoxicity mechanism of geldanamycin. For tanespimycin, a structurally similar molecule to geldanamycin, PELSA identified PRDX5 and NQO1 as shared off-target proteins. This result is consistent with previous report that NQO1 is involved in the metabolism of ansamycin HSP90 inhibitors (Reigan P D, et al., Molecular Pharmacology, 2011, 79(5): 823-832). Additionally, PELSA identified two previously-unreported off-target proteins of ganetespib, namely MAT2A and AKR1C2 (FIG. 10B). To further validate the binding between these two proteins and ganetespib, both proteins were purified, and a thermal shift assay was conducted to assess their interactions with ganetespib. As depicted in FIG. 10D, the thermal melting temperatures of both MAT2A and AKR1C2 significantly increased upon the addition of ganetespib, confirming the binding of AKR1C2 and MAT2A with ganetespib. Furthermore, the thermal shift assay results demonstrate that geldanamycin can induce changes in the energy states of AKR1C2, albeit with a lower stabilizing effect compared to ganetespib (FIG. 10D). Consistently, the PELSA results also indicated a smaller stabilizing effect of geldanamycin on AKR1C2 compared to ganetespib (FIG. 10B).

These findings clearly demonstrate the capability of PELSA coupled with dimethyl labeling to precisely detect proteins exhibiting altered energy states and effectively distinguish target proteins among structurally-similar and distinct inhibitors. The unknown target proteins identified by PELSA were also successfully validated using thermal shift assay with purified proteins. The identification of off-targets offers valuable insights into understanding the hepatotoxicity of ansamycin HSP90 inhibitors and exploring novel applications of ganetespib.

### Example 12

PELSA evaluation of the local affinity between heat shock protein inhibitors and their target proteins.

The procedures and conditions are the same as in Example 11, with the following modifications: 14 aliquots, each containing 50 µL HeLa cell lysate, were divided into experimental and control groups, with 7 aliquots in each group. In the experimental group, different concentrations of geldanamycin are added to the lysate achieve final concentrations of 100 µM, 10 µM, 1 µM, 100 nM, 10 nM, 1 nM, and 0.1 nM (geldanamycin dissolved in DMSO), while the samples in control group received an equal volume of DMSO. The treatment procedures for tanespimycin and ganetespib followed the same protocol as that for geldanamycin.

FIG. 11A illustrates that, using ganetespib as an example, only the peptides within the N-terminal domain of HSP90 proteins show an increase in change of peptide abundance with increasing drug concentration, eventually reaching a plateau. The affinities between HSP90 proteins and three HSP90 protein inhibitors were then calculated based on fold change of these peptides and the varying drug concentrations. The peptides with at least 12 quantification values (a total of 14 quantification values) were fitted to a four-parameter logistic equation using Prism software (GraphPad): Y=Bottom+(Top-Bottom)/(1+10^(LogEC50-X)*HillSlope)). The quality of fit between the raw data to this equation is evaluated using the Pearson correlation coefficient (R2), and peptides with a high fit quality (R2 > 0.9) were selected as candidate peptides. Additionally, the abundance change magnitude of candidate peptides at the highest ligand concentration should be at least 30% or higher. The median fold change of all qualified peptides from the same protein is considered as the protein's fold change at a specific ligand concentration, corresponding to the Y value in the four-parameter logistic equation. X still denotes the ligand concentration. Once again, Prism software is employed to fit the four-parameter logistic equation and determine the EC50 (FIG. 11B). FIG. 11C demonstrates the utilization of purified proteins in microscale thermophoresis (MST) to evaluate the affinity (Kd) between the three inhibitors and HSP90AA1. The affinity values obtained through MST align closely with the EC50s calculated using PELSA.

These findings demonstrate that the affinity values determined by PELSA are in agreement with conventional methods for determining affinity, such as microscale thermophoresis (MST) employed in this example. Moreover, this approach enables the acquisition of affinity data at the peptide level, which will contribute to understanding the interaction between ligands and specific protein regions.

## Claims

1. , A method for detecting change in the energy state of a protein, comprising the following steps:
A. Contacting two or more groups of protein solutions, each representing different energy states (one group serving as a control in original energy state, and the others as experimental groups with altered energy states), with a protease at a weight ratio of protease to total protein range from 1:1 to 1:50, and incubating for 0.5-60 minutes to enable digesting protein structures even in low energy states (i.e., digestion of proteins in a disruptive manner), which results in the direct generation of small peptides (molecular weight < 5 kDa) that contain two cleavage sites reflecting protein's local stability and are suitable for bottom-up (identifying proteins with digested peptides) mass spectrometry analysis.
B. Separating the small peptides (< 5-10 kDa) from the larger protein fragments (≥ 10 kDa).
C. Utilizing quantitative proteomic techniques and software (i.e., mass spectrometry quantification and analysis) to determine the abundance of small peptides (molecular weight < 5 kDa), to identify the proteins to which they belong to, and to pinpoint their positions on the proteins.
D. Determining the protein undergoing change in energy state by analyzing the abundance differences of the small peptides from the protein (< 5 kDa) between the experimental and control samples.
E. Determining the protein region undergoing change in energy state by analyzing the positions of the abundance-altered small peptides (< 5 kDa) within their corresponding protein.

2. , The method of claim 1, wherein the different energy states of the proteins include but are not limited to changes in energy state caused by one or more of the following:
A. Interacting with a ligand (including a drug, a metabolite in humans or animal, a plant extract, a nucleic acid, a metal ion, a peptide, an antibody, one or more proteins) resulting in change in protein energy state.
or B. Post-translational modification of the protein leading to the change in protein energy state.
or C. One or more of the following factors induced alterations in protein energy state: thermal **stimulation,** osmotic pressure changes, denaturing agent stimulation, oxidative stress, or disease.

3. ,A method for detecting the affinities between a ligand and a protein, comprising the following steps:
A. Contacting several aliquots of a protein solution with a ligand with a series of different final concentrations (may include a blank point without the ligand as a control group, and the others as experimental groups), respectively;
B. Incubating the above samples with a protease under non-denaturing conditions for 0.5-60 minutes, with an enzyme-to-protein weight ratio ranging from 1:1 to 1:50, to enable digesting protein structures even in low energy states (i.e., digestion of proteins in a disruptive manner), which results in the direct generation of a large number of small peptides (molecular weight < 5 kDa) that contain two cleavage sites reflecting protein's local stability and are suitable for bottom-up mass spectrometry analysis;
C. Separating the small peptides (molecular weight < 5-10 kDa) suitable for mass spectrometry detection from the large protein fragments (molecular weight >= 10 kDa).
D. Utilizing quantitative proteomic techniques and software (i.e., mass spectrometry quantification and analysis) to quantify and analyze the abundance of the small peptides (molecular weight < 5 kDa), identifying the corresponding proteins and determining the position of the small peptides on the proteins.
E. Calculating the local affinity between the proteins and the ligand, represented as the half-maximal effective concentration (EC50), based on the abundance changes of the small peptides in samples treated with different ligand concentrations, where the calculation is performed using a four-parameter logarithmic equation: Y = Bottom + (Top - Bottom) / (1 + 10^(LogEC50 - X) * Hill Slope), where in the equation Y represents the fold change in peptide abundance between the experimental and control groups, X represents the different ligand concentrations, Bottom and Top are the plateau values at the lower and upper ends of the four-parameter logarithmic equation curve, respectively (in the same unit as Y), Hill Slope refers to the absolute value of the maximum slope of the curve at the midpoint, and EC50 is the half-maximal effective concentration to be calculated; A smaller EC50 indicates a stronger affinity between the ligand and the protein region to which the peptide belongs to;
The points of ligand concentration should be at least 3, preferably more than 5.

4. , The method of claim 1, wherein the ligand can be one or more of the following substances: a drug, a metabolite from animal or plant organism, plant extract, a nucleic acid, a metal ion, a peptide, an antibody, a protein, or other substances that can interact with proteins.

5. , The method of claim 1 or 3, wherein the protein solution comprises either purified protein solution containing one protein or protein mixture containing two or more proteins; The protein mixture may originate from cells or tissue extracts of human, animals, plants, or bacteria; Protein extraction should be performed using gentle extraction methods, including but not limited to freeze-thaw cycles (freeze with liquid nitrogen and thaw in a water bath), liquid nitrogen grinding extraction, or homogenization extraction; For the extraction buffer, surfactants or denaturing agents that can disrupt protein conformation should be avoided to maintain the original conformations of proteins; The proteins to be tested can exist in the free form and/or in the immobilized form.

6. , The method of claim 1, wherein the proteins with different energy states, under their respective conformations, are subjected to the same digestion conditions; A large amount of protease is added to the protein solutions, initiating the digestion of protein structures in high-energy states, followed by the exposure of protein structures in low-energy states; The large amount of protease further leads to the subsequent digestion of structures in low-energy states and thus enables the generation of small peptides (molecular weight < 5 kDa) suitable for bottom-up mass spectrometry identification (identifying proteins with their digested peptides); Simultaneously, this digestion should result in different degrees of cleavage for proteins in different energy states; Trypsin, glutamyl endopeptidase, thermolysin, proteinase K, and one or more proteases that are specific or unspecific to substrates, can be used; The weight ratio of enzyme to protein should be between 1:1 and 1:50, preferably 1:1 to 1:10 when a protease inhibitor is present at a volume fraction of 1% to 2%; The digestion time should be adjusted within the range of 0.5 to 60 minutes; The digestion temperature should be determined based on the optimal temperatures required to maintain protease activity.

7. , The method of claim 3, wherein the ligand-treated group, comprising multiple protein solutions treated with various ligand concentrations, and the control group are subjected to the same digestion conditions; A large amount of protease is added to the protein solutions, initiating the digestion of protein structures in high-energy states, followed by the exposure of protein structures in low-energy states; The large amount of protease further leads to the subsequent digestion of structures in low-energy states and thus enables the generation of small peptides (molecular weight < 5 kDa) suitable for bottom-up mass spectrometry identification (identifying proteins with their digested peptides); Simultaneously, this digestion should result in different degrees of cleavage for proteins bound to ligands and those without ligands; Trypsin, glutamyl endopeptidase, thermolysin, proteinase K, and one or more proteases that are specific or unspecific to substrates, can be used; The weight ratio of enzyme to protein should be between 1:1 and 1:50, preferably 1:1 to 1:10 when a protease inhibitor is present at a volume fraction of 1% to 2%; The digestion time should be adjusted within the range of 0.5 to 60 minutes; The digestion temperature should be determined based on the optimal temperatures required to maintain protease activity.

8. , The method of claim 1 or claim 3, wherein the separation of small peptides from larger protein fragments can be achieved through various techniques, including but not limited to utilizing the differences in molecular weight, hydrophobicity, and thermal stability.

9. , The method of claim 1 or claim 3, wherein the quantitative proteomics technique refers to the quantitative analysis of the cleaved peptides in a bottom-up mass spectrometry analysis mode (identifying proteins with their digested peptides); The mass spectrometry data acquisition mode can be data-dependent acquisition (DDA) and data-independent acquisition (DIA); the quantification method can be label-free quantification and/or labeling-based quantification; The labeling-based quantification can be dimethyl labeling quantification, iTRAQ labeling quantification, TMT labeling quantification, TMTpro labeling quantification, or a combination thereof.
Software analysis refers to importing the raw mass spectrometry spectra into software to analyze and obtain quantitative information of small peptides, the protein information to which the small peptides belong to, and the position information of the small peptides on the protein; The software used for this analysis can be Maxquant (Cox, Germany), MSFragger (Alexey, USA), Spectronaut (Biognosys, Switzerland), or a combination thereof.

10. , The method of claim 1 or claim 3, wherein the method for determination of proteins with altered energy states is based on one or any combination of the following factors: significance of the differences in peptide abundance represented by -log10P values of the peptides, the magnitudes of the difference in peptide abundance represented by |log2 fold change| of the peptides, the number of differential peptides, and whether the fold change of peptides shows dose-dependent changes with the ligand concentrations;
Wherein the significance of difference in peptide abundance can be determined by performing significance tests, such as Empirical Bayes t-test or Student's t-test; The obtained P values could be used as the index for significance of peptide abundance difference between control and experimental groups; Peptides with -log10P value > 2~5 could be considered as indicative of significant differences between the experimental and control groups, suggesting proteins with altered energy states;
Wherein the magnitude of difference in peptide abundance (i.e., fold change) can be directly determined using the intensity ratio of the peptides between experimental group and control group, or through statistical tests such as Empirical Bayes t-test or Student's t-test; Proteins containing peptides with |log2 fold change| > 0.3~3 can be considered to have altered energy states;
Wherein the factor using the number of differential peptides includes the following steps: Sorting all peptides within each protein based on their significance of difference or magnitude of difference; the peptide with the highest significance of difference or magnitude of difference is defined as the top-ranked changed peptide for that protein; The second-ranked peptide represents peptide with the second highest significance of difference or magnitude of difference, followed by the third and fourth-ranked peptides; The -log10P values and log2 fold change of the top-ranked, second-ranked, or third and fourth-ranked peptide is considered as the protein's quantitative value; Proteins with -log10P value > 2~3 or |log2 fold change| > 0.3~3 are considered to have undergone changes in energy state; Proteins identified with altered energy states could contain one, two, three, or four peptides that meet the criteria of -log10P value > 2~3 or |log2 fold change| > 0.3~3;
Where in the factor by assessing whether the fold change of peptide abundance exhibits dose-dependent changes with increasing ligand concentrations include the following steps: The fold change of peptides obtained through the aforementioned method, at different ligand concentrations, along with the corresponding ligand concentrations, are imported to fit a four-parameter logarithmic equation; The goodness of fit between the original data and the equation is evaluated using the Pearson correlation coefficient R2; An R² value within the range of 0.75 to 0.95 indicates that the peptide exhibits dose-dependent changes with increasing ligand concentration, suggesting that the protein to which the peptide belongs is bound by the ligand. Combining two or more factors refers to meeting any two or more of the aforementioned criteria, or combining -log10P value, |log2 fold change|, N (number of differential peptides), and R2 in any proportion to generate a comprehensive scoring system.
